# EUROPEAN PATENT SPECIFICATION

(11) **EP 2 834 372 B1**
(45) Date of publication and mention of the grant of the patent: **27.09.2017**
(21) Application number: 13717220.1
(22) Date of filing: 04.04.2013
(51) Int. Cl.: C12Q 1/68

(54) **COMPLEX SETS OF MIRNAS AS NON-INVASIVE BIOMARKERS FOR EARLY DIAGNOSIS OF ACUTE MYOCARDIAL INFARCTION**
KOMPLEXE SÄTZE VON MIRNAS ALS NICHTINVASIVE BIOMARKER ZUR FRÜHEN DIAGNOSE EINES AKUTEN MYOKARDINFARKTS
ENSEMBLES COMPLEXES DE MIARN EN TANT QUE BIOMARQUEURS NON INVASIFS POUR LE DIAGNOSTIC PRÉCOCE DE L'INFARCTUS DU MYOCARDE AIGU

(30) Priority: 04.04.2012 EP 12163180
(43) Date of publication of application: 11.02.2015
(73) Proprietor: Hummingbird Diagnostics GmbH, 69120 Heidelberg (DE)
(72) Inventor: KELLER, Andreas, 66346 Püttlingen (DE); MEDER, Benjamin, 69221 Dossenheim (DE); VOGEL, Britta, 69120 Heidelberg (DE); KATUS, Hugo, 69120 Heidelberg (DE); BEIER, Markus, 69469 Weinheim (DE)
(74) Representative: Geling, Andrea
(86) International application number: PCT/EP2013/057118
(87) International publication number: WO 2013/150105

(56) References cited:
- WO-A1-2011/131354
- BENJAMIN MEDER ET AL: "MicroRNA signatures in total peripheral blood as novel biomarkers for acute myocardial infarction", BASIC RESEARCH IN CARDIOLOGY, STEINKOPFF-VERLAG, DA, vol. 106, no. 1, 1 October 2010 (2010-10-01), pages 13-23, XP019855605, ISSN: 1435-1803, DOI: 10.1007/S00395-010-0123-2
- Y. D'ALESSANDRA ET AL: "Circulating microRNAs are new and sensitive biomarkers of myocardial infarction", EUROPEAN HEART JOURNAL, vol. 31, no. 22, 9 June 2010 (2010-06-09), pages 2765-2773, XP055032528, ISSN: 0195-668X, DOI: 10.1093/eurheartj/ehq167
- REICHLIN TOBIAS ET AL: "Early Diagnosis of Myocardial Infarction with Sensitive Cardiac Troponin Assays.", NEW ENGLAND JOURNAL OF MEDICINE, vol. 361, no. 9, August 2009 (2009-08), pages 858-867, XP002679751, ISSN: 0028-4793
- T. ADACHI ET AL: "Plasma MicroRNA 499 as a Biomarker of Acute Myocardial Infarction", CLINICAL CHEMISTRY, vol. 56, no. 7, 1 July 2010 (2010-07-01), pages 1183-1185, XP055062403, ISSN: 0009-9147, DOI: 10.1373/clinchem.2010.144121
- C. Emilian ET AL: "MicroRNAs in Patients on Chronic Hemodialysis (MINOS Study)", Clinical Journal of the American Society of Nephrology, vol. 7, no. 4, 16 February 2012 (2012-02-16), pages 619-623, XP55253107, ISSN: 1555-9041, DOI: 10.2215/CJN.10471011
- Asli Tanindi ET AL: "Troponin elevation in conditions other than acute coronary syndromes", Vascular Health and Risk Management, 1 September 2011 (2011-09-01), page 597, XP55253121, DOI: 10.2147/VHRM.S24509
- Yq Li ET AL: "Comparing the diagnostic values of circulating microRNAs and cardiac troponin T in patients with acute myocardial infarction", CLINICS, vol. 68, no. 1, 28 January 2013 (2013-01-28), pages 75-80, XP055253129, BR ISSN: 1807-5932, DOI: 10.6061/clinics/2013(01)OA12
- EMILIAN CRISTINA ET AL: "MicroRNAs in patients on chronic hemodialysis (MINOS study).", CLINICAL JOURNAL OF THE AMERICAN SOCIETY OF NEPHROLOGY : CJASN APR 2012, vol. 7, no. 4, April 2012 (2012-04), pages 619-623, ISSN: 1555-905X
- TANINDI ASLI ET AL: "Troponin elevation in conditions other than acute coronary syndromes.", VASCULAR HEALTH AND RISK MANAGEMENT 2011, vol. 7, 2011, pages 597-603, ISSN: 1178-2048
- LI YING-QING ET AL: "Comparing the diagnostic values of circulating microRNAs and cardiac troponin T in patients with acute myocardial infarction.", CLINICS (SÃO PAULO, BRAZIL) JAN 2013, vol. 68, no. 1, January 2013 (2013-01), pages 75-80, ISSN: 1980-5322

### SEQUENCE LISTING

<110> febit holding GmbH
<120> Complex sets of miRNAs as non-invasive biomarkers for early diagnosis of acute myocardial infarction
<130> FP-012
<160> 9
<170> PatentIn version 3.5
<210> 1
   <211> 22
   <212> RNA
   <213> Homo sapiens
<400> 1
   caacaaauca cagucugcca ua 22
<210> 2
   <211> 19
   <212> RNA
   <213> Homo sapiens
<400> 2
   gggcgccugu gaucccaac 19
<210> 3
   <211> 21
   <212> RNA
   <213> Homo sapiens
<400> 3
   gcaguccaug ggcauauaca c 21
<210> 4
   <211> 24
   <212> RNA
   <213> Homo sapiens
<400> 4
   agccuggaag cuggagccug cagu 24
<210> 5
   <211> 22
   <212> RNA
   <213> Homo sapiens
<400> 5
   ugguugacca uagaacaugc gc 22
<210> 6
   <211> 23
   <212> RNA
   <213> Homo sapiens
<400> 6
   ugugcuugcu cgucccgccc gca 23
<210> 7
   <211> 24
   <212> RNA
   <213> Homo sapiens
<400> 7
   uggcccugac ugaagaccag cagu 24
<210> 8
   <211> 22
   <212> RNA
   <213> Homo sapiens
<400> 8
   aaccaucgac cguugagugg ac 22
<210> 9
   <211> 20
   <212> RNA
   <213> Homo sapiens
<400> 9
   ccccagggcg acgcggcggg 20

## Description

### TECHNICAL FIELD OF THE INVENTION

The present invention relates to a method for differential diagnosis of acute myocardial infarction (AMI). Further, the present invention relates to the use of cardiac troponin in early diagnosis of AMI in subjects with chest pain.

### BACKGROUND OF THE INVENTION

Today, biomarkers play a key role in early diagnosis, risk stratification, and therapeutic management of various diseases. While progress in biomarker research has accelerated over the last 5 years, the clinical translation of disease biomarkers as endpoints in disease management and as the foundation for diagnostic products still poses a challenge.
MicroRNAs (miRNAs) are a new class of biomarkers. They represent a group of small noncoding RNAs that regulate gene expression at the posttranslational level by degrading or blocking translation of messenger RNA (mRNA) targets. MiRNAs are important players when it comes to regulate cellular functions and in several diseases..
So far, miRNAs have been extensively studied in tissue material. It has been found that miRNAs are expressed in a highly tissue-specific manner. Disease-specific expression of miRNAs have been reported in many human cancers employing primarily tissue material as the miRNA source. In this context miRNAs expression profiles were found to be useful in identifying the tissue of origin for cancers of unknown primary origin.
Since recently it is known that miRNAs are not only present in tissues but also in other body fluid samples, including human blood. Nevertheless, the mechanism why miRNAs are found in body fluids, especially in blood, or their function in these body fluids is not well understood yet.

Various miRNA biomarkers found in tissue material have been proposed to be correlated with certain diseases, e.g. cancer. However, there is still a need for novel miRNAs as biomarkers for the detection and/or prediction of these and other types of diseases. Especially desirable are non-invasive biomarkers, that allow for quick, easy and cost-effective diagnosis/prognosis which cause only minimal stress for the patient eliminating the need for surgical intervention

Particularly, the potential role of miRNAs as non-invasive biomarkers for the early diagnosis and/or differential diagnosis of AMI has not been systematically evaluated yet. In addition, many of the miRNA biomarkers presently available for diagnosing and/or prognosing of diseases have shortcomings such as reduced sensitivity, not sufficient specificity or do not allow timely diagnosis or represent invasive biomarkers. Accordingly, there is still a need for novel and efficient miRNAs or sets of miRNAs as biomarkers, effective methods and kits for the non-invasive early diagnosis and/or differential diagnosis of diseases such as AMI.

BENJAMIN MEDER et al. (Basic Research in Cardiology, Steinkopff-Verlag, DA, Vol. 106, No. 1, October 1, 2010, pages 13 to 23) relate to miRNA signatures in total peripheral blood as novel biomarkers for acute myocardial infarction.

ASLI TANINDI et al. (Vascular Health and Risk Management, September 1, 2011, page 597) refer to troponin elevation in conditions other than acute coronary syndromes.

C. EMILIAN et al. (Clinical Journal of the American Society of Nephrology, Vol. 7, No. 4, February 16, 2012, pages 619 to 623) disclose miRNAs in patients on chronic hemodialysis (MINOS Study).

The inventors of the present invention assessed for the first time the expression of miRNAs on a whole-genome level in subjects with acute myocardial infarction (AMI) as non-invasive biomarkers from body fluids, preferably in blood, more preferably in blood cells. They surprisingly found that miRNAs are significantly dysregulated in blood, preferably in blood cells of AMI subjects at an early stage in comparison to healthy controls and/or dilated cardiomyopathy patients and thus, miRNAs are appropriated non-invasive biomarkers for early diagnosis and/or differential diagnosis of AMI. Furthermore, the inventors surprisingly found that the dysregulation of miRNAs is found at an earlier time point compared to cardiac Troponins, currently being the gold-standard for diagnosis of AMI. Therefore, the inventors of the invention identified for the first time miRNAs as non-invasive surrogate biomarkers for early diagnosis of AMI that may complement existing Troponin tests. The inventors of the present invention identified single miRNAs which diagnose AMI with high specificity, sensitivity and accuracy at an early time point. The inventors of the present invention also pursued a multiple biomarker strategy, thus implementing sets of miRNA biomarkers for early diagnosing AMI leading to added specificity, sensitivity, accuracy and predictive power, thereby circumventing the limitations of single biomarker. In detail, they identified unique sets of miRNAs (miRNA signatures) that allow for non-invasive early diagnosis of AMI with even higher power, indicating that sets of miRNAs (miRNA signatures) derived from a body fluid sample, such as blood, preferably a blood cell containing sample from a subject (e.g. human) can be used as novel non-invasive biomarkers for early diagnosis and/or differential diagnosis of AMI.

### SUMMARY OF THE INVENTION

In a first aspect, the invention provides a method for differential diagnosis of acute myocardial infarction (AMI), comprising the steps :
(a) determining the level of at least one miRNA with SEQ ID NO: 5 (hsa-miR-380*) in a blood cell test sample from a subject, particularly a human subject,
(b) comparing the level of said at least one miRNA of (a) with a reference level of said at least one miRNA,
(c) determining the level of cardiac Troponin in a serum or plasma test sample from said subject, and
(d) comparing the level of cardiac Troponin of (c) with a reference level of cardiac Troponin,
   wherein the comparison of step (b) together with the comparison of step (d) allows for differential diagnosis of acute myocardial infarction and diseases that give rise to cardiac Troponin levels and that are not acute myocardial infarction,
   wherein the cardiac Troponin level being above the 99th percentile value of a reference population and the level of said at least one miRNA being not decreased by a factor of at least 1.5 results in the diagnosis of diseases that give rise to cardiac troponin levels and that are not acute myocardial infarction,
   wherein the cardiac Troponin level being above the 99th percentile value of a reference population and the level of said at least one miRNA being decreased by a factor of at least 1.3 results in the diagnosis of acute myocardial infarction, or wherein the cardiac Troponin level being not above the 99th percentile value of a reference population and the level of said at least one miRNA being decreased by a factor of at least 1.3 results in the diagnosis of acute myocardial infarction.

In a second aspect, the invention provides the use of cardiac Troponin in early diagnosis of acute myocardial infarction (AMI) in subjects with chest pain characterized by a cardiac Troponin level being above the 99th percentile value of a reference population, preferably above 14pg/ml, more preferably above 50 pg/ml and by a not decreased level of a factor of at least 1.5, preferably by a not decreased level of a factor of at least 1.3 of at least one miRNA in a blood cell test sample from said subject, wherein said at least one miRNA is selected from SEQ ID NO: 5 (hsa-miR-380*).

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*" refer to single-stranded RNA molecules of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used. Described herein are (target) miRNAs which are dysregulated in biological samples such as blood or blood cells, of AMI patients in comparison to healthy controls and/or dilated cardiomyopathy (DCM) patients. Said (target) miRNAs are preferably selected from the group consisting of SEQ ID NO: 1 to 9.

The term "miRBase" refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

As used herein, the term "nucleotides" refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.
As described herein, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. Described herein are polynucleotides in form of single polynucleotide strands as probes for binding to, hybridizing with or detecting complementary sequences of (target) miRNAs for early diagnosing and/or differential diagnosing of AMI. Said (target) miRNAs described herein are preferably selected from the group consisting of SEQ ID NO: 1 to 9. Additionally, (a) polynucleotide(s) may be employed as a primer(s) in PCR-type reactions for detecting (a) miRNA(s).

Because of the conservation of miRNAs among species, for example between humans and other mammals, e.g. animals such as mice, monkey or rat, the polynucleotide(s) described herein may not only be suitable for detecting a miRNA(s) of a specific species, e.g. a human miRNA, but may also be suitable for detecting the respective miRNA orthologue(s) in another species, e.g. in another mammal, e.g. animal such as mouse or rat.

The term "sensitivity", as used herein, means a statistical measure of how well a binary classification test correctly identifies a condition, for example how frequently it correctly classifies a heart and cardiovascular system disease into the correct type out of two or more possible types/alternatives (e.g. heart and cardiovascular system disease type and healthy type). The sensitivity for class A is the proportion of cases that are determined to belong to class "A" by the test out of the cases that are in class "A". A theoretical, optimal prediction can achieve 100% sensitivity (i.e. predict all patients from the sick group as sick).

The term "specificity", as used herein, means a statistical measure of how well a binary classification test correctly identifies a condition, for example how frequently it correctly classifies a heart and cardiovascular system disease into the correct type out of two or more possible types/alternatives. The specificity for class A is the proportion of cases that are determined to belong to class "not A" by the test out of the cases that are in class "not A". A theoretical, optimal prediction can achieve 100% specificity (i.e. not predict anyone from the healthy group as sick).

The term "accuracy", as used herein, means a statistical measure for the correctness of classification or identification of sample types. The accuracy is the proportion of true results (both true positives and true negatives).

The term "biological sample", as used herein, refers to any biological sample containing miRNA(s). Said biological sample may be a biological fluid, tissue, cell(s) or mixtures thereof. For example, biological samples described herein are body fluids, tissue (e.g. section or explant) samples, cell culture samples, cell colony samples, single cell samples, collection of single cell samples, blood samples (e.g. whole blood or a blood fraction such as serum or plasma), urine samples, or samples from other peripheral sources. Said biological samples may be mixed or pooled, e.g. a biological sample may be a mixture of blood and urine samples. A "biological sample" may be provided by removing cell(s), cell colonies, an explant, or a section from a subject suspected to be affected by AMI, but may also be provided by using a previously isolated sample. For example, a tissue sample may be removed from a subject suspected to be affected by AMIs by conventional biopsy techniques or a blood sample may be taken from a subject suspected to be affected by AMI by conventional blood collection techniques. The biological sample, e.g. tissue or blood sample, may be obtained from a subject suspected to be affected by AMI prior to initiation of the therapeutic treatment, during the therapeutic treatment and/or after the therapeutic treatment.

The term "body fluid sample", as used herein, refers to liquids originating from the body of a subject. Said body fluid samples include, but are not limited to, blood, urine, sputum, breast milk, cerebrospinal fluid, amniotic fluid, bronchial lavage, colostrum, seminal fluid, cerumen (earwax), endolymph, perilymph, gastric juice, mucus, peritoneal fluid, pleural fluid, saliva, sebum (skin oil), semen, sweat, tears, vaginal secretion, vomit including components or fractions thereof Said body fluid samples may be mixed or pooled, e.g. a body fluid sample may be a mixture of blood and urine samples or blood and tissue material. A "body fluid sample" may be provided by removing a body liquid from a subject, but may also be provided by using previously isolated sample material.
Preferably, the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.
Said "body fluid sample" allows for a non-invasive diagnosis/and or prognosis of a subject.

The term "blood sample", as used herein, refers to a blood sample originating from a subject. The "blood sample" may be derived by removing blood from a subject by conventional blood collecting techniques, but may also be provided by using previously isolated and/or stored blood samples. For example a blood sample may be whole blood, plasma, serum, PBMC (peripheral blood mononuclear cells), blood cellular fractions including red blood cells (erythrocytes), white blood cells (leukocytes), platelets (thrombocytes), or blood collected in blood collection tubes (e.g. EDTA-, heparin-, citrate-, PAXgene- , Tempus-tubes) including components or fractions thereof. For example, a blood sample may be taken from a subject suspected to be affected or to be suspected to be affected by AMI, prior to initiation of a therapeutic treatment, during the therapeutic treatment and/or after the therapeutic treatment.
Preferably, the blood sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.
Said "body fluid sample" allows for a non-invasive diagnosis/and or prognosis of a subject.

Preferably, when the blood sample is collected from the subject the RNA-fraction, especially the the miRNA fraction, is guarded against degradation. For this purpose special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction and/or the miRNA fraction are employed.

The biological sample, preferably the body fluid sample may be from a subject (e.g. human or mammal) that has been therapeutically treated or that has not been therapeutically treated. In one embodiment, the therapeutical treatment is monitored on the basis of the detection of the miRNA or set of miRNAs by the polynucleotide or set of polynucleotides described herein. It is also preferred that total RNA or a subfraction thereof, isolated (e.g. extracted) from a biological sample of a subject (e.g. human or animal), is used for detecting the miRNA or set of miRNAs by the polynucleotide or set of polynucleotides or primer pairs described herein.

In the context of the present invention, the biological sample is a blood cell sample.

The term "non-invasive", as used herein, refers to methods for obtaining a biological sample, particularly a body fluid sample, without the need for an invasive surgical intervention or invasive medical procedure. A blood drawn represents a non-invasive procedure, therefore a blood-based test (utilizing blood or fractions thereof) is a non-invasive test. Other body fluid samples for non-invasive tests are e.g. urine, sputum, tears, mothers mild, cerumen, sweat, saliva, vaginal secretion, vomit, etc..

The term "biomarker", as used herein, represents a characteristic that can be objectively measured and evaluated as an indicator of normal and disease processes or pharmacological responses. A biomarker is a parameter that can be used to measure the onset or the progress of disease or the effects of treatment. The parameter can be chemical, physical or biological.

The term "diagnosis" as used herein refers to the process of determining a possible disease or disorder and therefore is a process attempting to define the (clinical) condition of a subject. The determination of the expression level of a set of miRNAs described herein correlates with the (clinical) condition of a subject. Preferably, the diagnosis comprises (i) determining the occurrence/presence of AMI, (ii) monitoring the course of AMI, (iii) staging of AMI, (iv) measuring the response of a patient with AMI to therapeutic intervention, and/or (v) segmentation of a subject suffering from AMI.

The term "prognosis" as used herein refers to describing the likelihood of the outcome or course of a disease or a disorder. Preferably, the prognosis comprises (i) identifying of a subject who has a risk to develop AMI, (ii) predicting/estimating the occurrence, preferably the severity of occurrence of AMI, and/or(iii) predicting the response of a subject with AMI to therapeutic intervention.

The term "miRNA expression profile" as used herein, represents the determination of the miRNA expression level or a measure that correlates with the miRNA expression level in a biological sample. The miRNA expression profile may be generated by any convenient means, e.g. nucleic acid hybridization (e.g. to a microarray, bead-based methods), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX, Firefly Bioworks) and the like, that allow the analysis of differential miRNA expression levels between samples of a subject (e.g. diseased) and a control subject (e.g. healthy, reference sample, non-AMI patients). The sample material measured by the aforementioned means may be total RNA, labeled total RNA, amplified total RNA, cDNA, labeled cDNA, amplified cDNA, miRNA, labeled miRNA, amplified miRNA or any derivatives that may be generated from the aforementioned RNA/DNA species. By determining the miRNA expression profile, each miRNA is represented by a numerical value. The higher the value of an individual miRNA, the higher is the expression level of said miRNA, or the lower the value of an individual miRNA, the lower is the expression level of said miRNA.
The "miRNA expression profile", as used herein, represents the expression level/expression data of a single miRNA or a collection of expression levels of at least two miRNAs, preferably of least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more, or up to all known miRNAs.

The term "differential expression" of miRNAs as used herein, means qualitative and/or quantitative differences in the temporal and/or local miRNA expression patterns, e.g. within and/or among biological samples, body fluid samples, cells, blood cells, or within blood. Thus, a differentially expressed miRNA may qualitatively have its expression altered, including an activation or inactivation in, for example, blood from a diseases subject versus blood from a healthy subject. The difference in miRNA expression may also be quantitative, e.g. in that expression is modulated, i.e. either up-regulated, resulting in an increased amount of miRNA (increased level of miRNA), or down-regulated, resulting in a decreased amount of miRNA (decreased level of miRNA). The degree to which miRNA expression differs need only be large enough to be quantified via standard expression characterization techniques, e.g. by quantitative hybridization (e.g. to a microarray, to beads), amplification (PCR, RT-PCR, qRT-PCR, high-throughput RT-PCR), ELISA for quantitation, next generation sequencing (e.g. ABI SOLID, Illumina Genome Analyzer, Roche 454 GS FL), flow cytometry (e.g. LUMINEX) and the like.

Nucleic acid hybridization may be performed using a microarray/biochip or *in situ* hybridization. *In situ* hybridization is preferred for the analysis of a single miRNA or a set comprising a low number of miRNAs (e.g. a set of at least 1 to 9 miRNAs such as a set of 1, 2, 3, 4, 5, 6, ,7 ,8 or 9 miRNAs). The microarray/biochip, however, allows the analysis of a single miRNA as well as a complex set of miRNAs (e.g. a all known miRNAs or subsets therof).
For nucleic acid hybridization, for example, the polynucleotides (probes) described herein with complementarity to the corresponding miRNAs to be detected are attached to a solid phase to generate a microarray/biochip (e.g. 9 polynucleotides (probes) which are complementary to the 9 miRNAs having SEQ ID NO: 1 to 9. Said microarray/biochip is then incubated with a biological sample containing miRNAs, isolated (e.g. extracted) from the body fluid sample such as blood sample from a subject such as a human or an animal, which may be labelled, e.g. fluorescently labelled, or unlabelled. Quantification of the expression level of the miRNAs may then be carried out e.g. by direct read out of a label or by additional manipulations, e.g. by use of a polymerase reaction (e.g. template directed primer extension, MPEA-Assay, RAKE-assay) or a ligation reaction to incorporate or add labels to the captured miRNAs.
Alternatively, the polynucleotides which are at least partially complementary (e.g.a set of chimeric polynucleotides with each a first stretch being complementary to a set of miRNA sequences and a second stretch complementary to capture probes bound to a solid surface (e.g. beads, Luminex beads)) to miRNAs having SEQ ID NO: 1 to 9 are contacted with the biological sample containing miRNAs (e.g a body fluid sample, preferably a blood sample, more preferably a blood cell sample) in solution to hybridize. Afterwards, the hybridized duplexes are pulled down to the surface (e.g a plurality of beads) and successfully captured miRNAs are quantitatively determined (e.g. FlexmiR-assay, FlexmiR v2 detection assays from Luminex).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 1 to 9 miRNAs such as a set of 1, 2, 3, 4, 5, 6, ,7 ,8 or 9 miRNAs), whereas high-throughput RT-PCR technologies (e.g. OpenArray from Applied Biosystems, SmartPCR from Wafergen, Biomark System from Fluidigm) are also able to measure large sets (e.g a set of 10, 20, 30, 50, 80, 100, 200 or more) to all known miRNAs in a high parallel fashion. RT-PCR is particularly suitable for detecting low expressed miRNAs.

The aforesaid real time polymerase chain reaction (RT-PCR) may include the following steps:
(i) extracting the total RNA from a biological sample or body fluid sample such as a blood sample (e.g. whole blood, a blood cell sample, serum, or plasma) of a subjects such as human or animal, and obtaining cDNA samples by RNA reverse transcription (RT) reaction using universal or miRNA-specific primers; or collecting a body fluid sample such as urine or blood sample (e.g. whole blood, serum, or plasma) of a patient such as human or animal, and conducting reverse transcriptase reaction using universal or miRNA-specific primers (e.g. looped RT-primers) within the body fluid sample such as urine or blood sample (e.g. whole blood, blood cell sample, serum, or plasma) being a buffer so as to prepare directly cDNA samples, (ii) designing miRNA-specific cDNA forward primers and providing universal reverse primers to amplify the cDNA via polymerase chain reaction (PCR), (iii) adding a fluorescent dye (e.g. SYBR Green) or a fluorescent probe (e.g. Taqman probe) probe to conduct PCR, and (iv) detecting the miRNA(s) level in the body fluid sample such as urine or blood sample (e.g. whole blood, blood cell sample, serum, or plasma).

A variety of kits and protocols to determine an expression profile by real time polymerase chain reaction (RT-PCR) such as real time quantitative polymerase chain reaction (RT qPCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI7300. Alternative kits are available from Ambion, Roche, Qiagen, Invitrogen, SABiosciences, Exiqon etc.

The term "subject", as used herein, means a patient or individual or mammal suspected to be affected by AMI. The patient may be diagnosed to be affected by AMI, i.e. diseased, or may be diagnosed to be not affected by AMI, i.e. healthy or another disease characterized by damage of the heart muscle that is not AMI. The subject may also be diagnosed to be affected by a specific form of AMI. The subject may further be diagnosed to develop AMI or a specific form of AMI as the inventors of the present invention surprisingly found that miRNAs representative for AMI are already present in the biological sample, e.g. blood sample, at an early stage of AMI. It should be noted that a subject that is diagnosed as being healthy, i.e. not suffering from AMI or from a specific form of AMI, may possibly suffer from another disease not tested/known. The subject may be any mammal, including both a human and another mammal, e.g. an animal such as a rabbit, mouse, rat, or monkey. Human subjects are particularly preferred. Therefore, the miRNA from a subject may be a human miRNA or a miRNA from another mammal, e.g. an animal miRNA such as a mouse, monkey or rat miRNA, or the miRNAs comprised in a set may be human miRNAs or miRNAs from another mammal, e.g. animal miRNAs such as mouse, monkey or rat miRNAs.

The term "control subject", as used herein, may refer to a subject known not to be affected with AMI (negative control), i.e. healthy or another disease characterized by damage of the heart muscle that is not AMI. It may also refer to a subject known to be effected by another disease/condition. It should be noted that a control subject that is known to be healthy, i.e. not suffering from AMI, may possibly suffer from another disease not tested/known. The control subject may be any mammal, including both a human and another mammal, e.g. an animal such as a rabbit, mouse, rat, or monkey. Human "control subjects" are particularly preferred. A reference (expression) level may be determined from a number of samples in a number of (control) subjects, preferably human subjects, not suffering from AMI. The reference level may be an average of a number of expression levels of (a) miRNA(s) determined in number of (control) subjects not suffering from AMI.

The term "acute myocardial infarction (AMI)" or myocardial infarction (MI) is commonly known as a heart attack. AMI results from the interruption of blood supply to a part of the heart, causing heart cells to die. This is most commonly due to occlusion (blockage) of a coronary artery following the rupture of a vulnerable atherosclerotic plaque, which is an unstable collection of lipids (cholesterol and fatty acids) and white blood cells (especially macrophages) in the wall of an artery. The resulting ischemia (restriction in blood supply) and ensuing oxygen shortage, if left untreated for a sufficient period of time, can cause damage or death (infarction) of heart muscle tissue (myocardium). Classical symptoms of acute myocardial infarction include sudden chest pain (typically radiating to the left arm or left side of the neck), shortness of breath, nausea, vomiting, palpitations, sweating. Among the diagnostic tests available to detect heart muscle damage are an electrocardiogram (ECG), echocardiography, cardiac MRI and various blood tests. The most often used blood markers are the creatine kinase-MB (CK-MB) fraction and the Troponin levels. Due to its cardio-specificity cardiac Troponins have become the key biomarker for detection of myocardial damage. In the clinical context, a myocardial infarction can be further subclassified into a ST elevation MI (STEMI) versus a non-ST elevation MI (non-STEMI) based on ECG changes. Most cases of STEMI (ST elevation MI) are treated with thrombolysis or percutaneous coronary intervention (PCI). NSTEMI (non-ST elevation MI) should be managed with medication, although PCI is often performed during hospital admission. Ischemic heart disease (which includes myocardial infarction, angina pectoris and heart failure when preceded by myocardial infarction) was the leading cause of death for both men and women worldwide in 2004.

The term "Troponin" refers to a complex of three regulatory proteins (Troponin C, Troponin I and Troponin T) that are integral to muscle contraction in skeletal and cardiac muscle, but not in smooth muscle. Certain subtypes of Troponin (cardiac Troponin I and T) are very sensitive and specific indicators of damage to the heart muscle (myocardium). They are measured in the blood (e.g in serum or plasma) to differentiate between unstable angina and acute myocardial infarction (AMI, heart attack) in patients with chest pain or acute coronary syndrome. A patient who had suffered from a myocardial infarction would have an area of damaged heart muscle and so would have elevated cardiac Troponin levels in the blood. It is important to note that cardiac Troponins are a marker of all heart muscle damage, not just myocardial infarction. Other conditions including, but not limited to myocarditis, pulmonary embolism, heart failure, (myo)pericarditis, cardiomyopathy, (left) ventricular hypertrophy, peripartum cardiomyopathy, Takotsubo cardiomyopathy, cardiac amyloidosis, non-ischemic (systolic) heart failure that directly or indirectly lead to heart muscle damage can also increase Troponin levels. A rise and/or fall of cardiac Troponin T or I with at least one value above the 99th percentile value of a reference population is today an integral part of the universal definition of myocardial infarction. However, Troponins do also have some inherent limitations as biomarkers for AMI. For instance, they reflect myocardial damage only with a certain delay since they need to be released to the blood stream from undergoing cardiomyocytes, hence this delay compromises their use as good indicators for early diagnosis of AMI

The term "Troponin test" or "cardiac Troponin test" refers to tests measuring the blood levels of cardiac Troponins, including cardiac Troponin T and Troponin I. The blood tests, measuring the cardiac Troponin T and Troponin I are currently carried out by immunoassay methods. Manufacturers of Troponin blood tests include Roche, Siemens, Abbott and others. In 5^{th} generation high sensitive Troponin assays the delay for detecting myocardial damage has been reduced. However, the reduction in the detection limit goes along with a decreased clinical specificity and elevated Troponins are not necessarily due to acute coronary syndromes but can be caused by other acute or chronic alterations of the cardiovascular system, such as pulmonary embolism, myocarditis, or heart failure. Accordingly, there is a need for an early and specific discrimination of acute coronary syndromes including AMI, also in the presence of other diseases that give rise to positive Troponin levels due to damage of the heart muscle. This need could further benefit from a multiple biomarker strategy, combining the diagnostic power of different biomarkers to circumvent these limitations.

Due to the shortcomings of current state of the art in diagnosis for AMI, there is an urgent need for better, non-invasive tests, especially for early diagnosis of AMI and differential diagnosis of AMI to other cardiovascular diseases with concomitant heart muscle damage to further diagnosis and prognosis options for patients. Especially, a multiple biomarker strategy, complementing the cardiac Troponin tests would be highly beneficial to patient treatment regime and the health care systems in general.

The inventors of the present invention surprisingly found that miRNAs are significantly dysregulated in blood, preferably in blood cell samples of AMI subjects in comparison to a cohort of controls (healthy subjects or dilated cardiomyopathy patients (DCM)) and thus, miRNAs are appropriated biomarkers for early diagnosis and/or differential diagnosis of AMI in a non-invasive fashion. Furthermore, the sets of miRNAs described herein lead to high performance in early diagnosis and/or differential diagnosis of AMI, thus expose very high specificity, sensitivity and accuracy. The inventors succeeded in determining the miRNAs that are differentially regulated in blood samples, preferably in blood cell samples, from patients having AMI compared to a cohort of controls (healthy subjects or DCM patients), even at an very early timepoint after onset of chest pain. (see experimental section for experimental details).

In addition to using the diagnostic power of single miRNA biomarkers the inventors of the present invention employed more than one miRNA biomarker, i.e. sets of miRNA biomarkers (signatures), to further increase and/or improve the performance for early and/or differential diagnosing AMI.

The inventors of the present invention surprisingly found that this approach yields in miRNAs or miRNA sets (signatures) that provide high diagnostic accuracy, specificity and sensitivity in the early and/or differential diagnosis of AMI in patients. Furthermore, the identified miRNAs or miRNA sets allow for differential diagnosis of AMI to other diseases characterized by heart muscle damage giving rise to positive cardiac Troponin tests. The inventors found that the identified biomarker are ideally suited to complement existing cardiac Troponin tests for earlier diagnosis and added specificity.

Described herein is a method for early diagnosis of AMI comprising the steps of:
(a) determining the level of at least one microRNA(s), e.g. 1, 2, 3,,4, 5, 6, 7, 8 or 9 miRNA(s), selected from the group consisting of SEQ ID NO: 1 to SEQ ID NO: 9 in a blood cell test sample from a subject, particularly a human subject,
(b) comparing the level of said miRNA(s) of step (a) with the reference level of said at least one miRNA(s)
   wherein a decrease of the level of said at least one miRNA(s) in the test sample in comparison to the reference level allows for early diagnosis of acute myocardial infarction

It is preferred that the level of miRNA with SEQ ID NO: 1, SEQ ID NO: 2, SEQ ID NO: 3, SEQ ID NO: 4, SEQ ID NO: 5, SEQ ID NO: 6 or SEQ ID NO: 7, a fragment thereof, or a sequence have at least 90% sequence identity thereto is determined. It is particularly preferred that the level of miRNA with SEQ ID NO: 1, SEQ ID NO: 2, or SEQ ID NO: 3, a fragment thereof, or a sequence have at least 90% sequence identity thereto is determined.

It is further preferred that the level of miRNAs with SEQ ID NO: 1, SEQ ID NO: 2, SEQ ID NO: 3, SEQ ID NO: 4, SEQ ID NO: 5, SEQ ID NO: 6 or SEQ ID NO: 7, fragments thereof, or sequences have at least 90% sequence identity thereto are determined. It is particularly preferred that the level of miRNAs with SEQ ID NO: 1, SEQ ID NO: 2, or SEQ ID NO: 3, fragments thereof, or a sequence have at least 90% sequence identity thereto are determined.

It is more preferred that the levels of at least 2 miRNAs having (i) SEQ ID NO: 1 and SEQ ID NO: 2, fragments thereof, or sequences have at least 90% sequence identity thereto, (ii) SEQ ID NO: 1 and SEQ ID NO: 3, fragments thereof, or sequences have at least 90% sequence identity thereto, (iii) SEQ ID NO: 1 and SEQ ID NO: 4, fragments thereof, or sequences have at least 90% sequence identity thereto, (iv) SEQ ID NO: 2 and SEQ ID NO: 3, fragments thereof, or sequences have at least 90% sequence identity thereto, (v) SEQ ID NO: 2 and SEQ ID NO : 4, fragments thereof, or sequences have at least 90% sequence identity thereto, (vi) SEQ ID NO: 3 and SEQ ID NO: 4, fragments thereof, or sequences have at least 90% sequence identity thereto are determined. It is further preferred that the level of at least 2 miRNAs, having (i) SEQ ID NO 1, SEQ ID NO 2 and SEQ ID NO: 3, fragments thereof, or sequences have at least 90% sequence identity thereto, (ii) SEQ ID NO: 1, SEQ ID NO: 2 and SEQ ID NO:4, fragments thereof, or sequences have at least 90% sequence identity thereto, (iii) SEQ ID NO 1, SEQ ID NO 3 and SEQ ID NO: 4, fragments thereof, or sequences have at least 90% sequence identity thereto, (iv) SEQ ID NO: 2, SEQ ID NO: 3 and SEQ ID NO:4, fragments thereof, or sequences have at least 90% sequence identity thereto are determined.

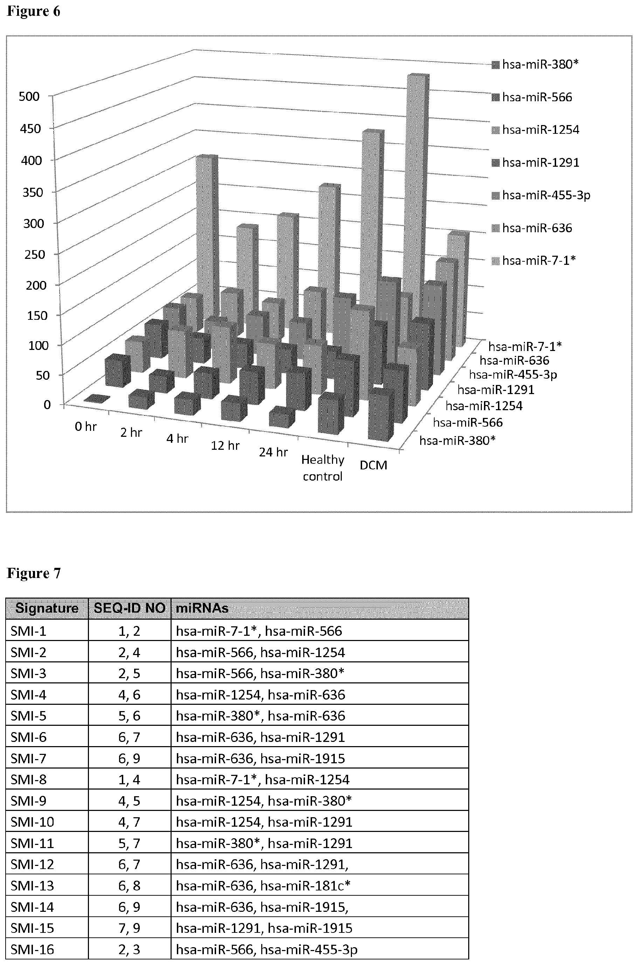

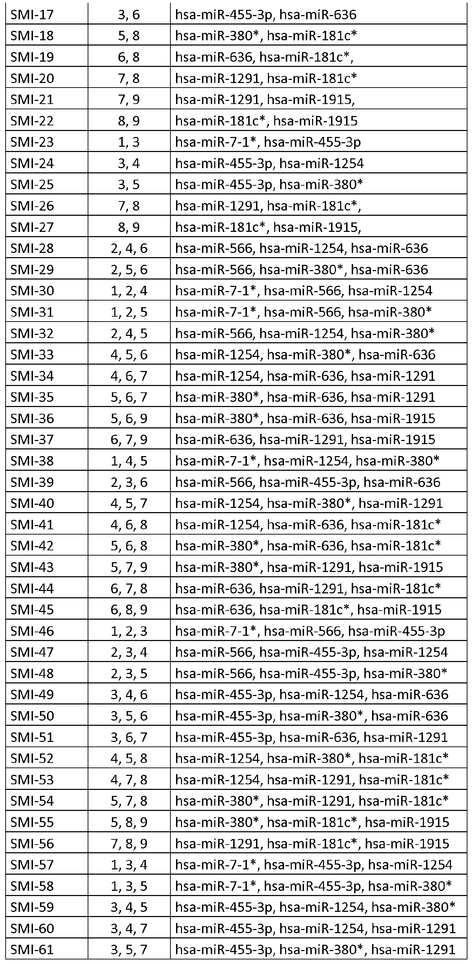

It is further preferred, that the levels of a set of miRNAs, listed in Figure 7, fragments thereof, or sequences have at least 90% sequence identity thereto, are determined. It is further preferred, that the levels of at least 2 miRNAs are selected from one or more sets listed in Figure 7 are determined.

The term "blood cell test sample", as used herein, refers to any biological sample that contains blood cells and (a) microRNA(s), preferably (a) miRNA(s) having SEQ ID NO: 1 to SEQ ID NO: 9, fragments thereof, or sequences have at least 90% sequence identity thereto. Said blood cell containing test sample may be a blood sample or any other biological sample that contains blood cells. Said test sample may be a mixed or a pooled sample, e.g. it may be a mixture of a serum and a whole blood sample, a mixture of plasma and whole blood sample, a mixture of a tissue and a serum sample, a mixture of a tissue and a plasma sample.
Preferably, a "blood cell test sample" is a whole blood sample, a PBMC sample, a blood cell culture sample, a sample containing red blood cells (erythrocytes), white blood cells (leukocytes), thrombocytes (platelets) or mixtures thereof. It is further preferred that the blood cell test sample contains defined blood cell subfractions (e.g. CD3, CD4, CD8, C14, CD 15, CD 56, T-helper cells, cytotoxic T cells, killer cells, natural killer cells, eosinophiles, neutrophils, basophiles, lymphocytes, monocytes, T cells, regulatory T cells, B cells, macrophages, dendritic cells, reticulocytes, thrombocytes, etc.) or mixtures thereof. It is particularly preferred, that the blood cell test sample is derived from whole blood collected in a PAXgene tube, a Tempus tube or in conventional blood collection tubes to which RNA-stabilization agents may be added.

It is further preferred, that the "blood cell test sample", has a volume of between 0.01 and 20 ml, more preferably between 0.1 and 10 ml, most preferably between 0.25 and 8 ml and most preferably between 0.5 and 4 ml, e.g. 0.1, 0.2, 0.3, 0.4, 0.5, 0.6, 0.7, 0.8, 0.9, 1, 1.5, 2, 2.5, 3, 3.5, 4, 5, 6, 7, 8, 9 ,10, 11, 12, 13, 14, 15, 16, 17, 18 ,19 or 20 ml.

Preferably the "blood cell test sample" is collected by a blood collection tube. It is preferred that the blood collection tube contains means for stabilizing the RNA-fraction, especially the small RNA-fraction including the miRNA fraction. Optionally the blood collection tube further contains means for cell lysis. Not limiting examples for blood collection tubes, already including means for RNA-stabilization are Tempus tubes (Applied Biosystems, Ambion) and tubes already including means for RNA-stabilization and cell lysis are PAXgene tubes (www.preanalytix.com). Optionally, RNA-stabilzation agents, e.g. RNAlater (Ambion), RNAretain (Asuragen), and/or cell lysis reagents can be added to conventional blood collection tubes (e.g. serum, plasma, EDTA-blood, Heparin-, Citrate-tubes).

The "blood cell test sample" may be derived from any organism or subject such as a vertebrate, particularly from a mammal, including human or other mammals (e.g. rodent, monkey, mouse, rat, rabbit, guinea pig, dog). Preferably the sample is derived from a human (subject). Preferably the (test)sample may be derived from a subject that is suspected to suffer from a cardiovascular disease, more preferably from a heart disease, even more preferably from acute coronary syndrome, most preferably the subject is suspected to suffer from acute myocardial infarction. Furthermore, the test sample may be derived from a subject that has/shows symptoms of chest pain.

It is further preferred that the "blood cell test sample" is isolated from a subject, preferably from a human subject, that suffers or is suspected to suffer from a cardiovascular disease, especially a disease that is characterized by damage of the heart muscle. Not limiting examples for such diseases include acute coronary syndrome, acute myocardial infarction (AMI), heart failure, cardiomyopathy, dilated cardiomyopathy, myocarditis, pulmonary embolism, stable angina, unstable angina. It is also preferred that the "blood cell test sample" is isolated from a subject or patient that shows symptoms of chest pain, especially subjects or patients presenting to a emergency unit or emergency department with chest pain.

The term "early diagnosis", as used herein, refers to an early point in time in respect to either onset of symptoms of chest pain or suspicion for a cardiovascular disease in a subject or patient, including but not limited to acute coronary syndrome, acute myocardial infarction (AMI), heart failure, cardiomyopathy, dilated cardiomyopathy, myocarditis, pulmonary embolism, stable angina, unstable angina. Preferably, "early diagnosis" refers to an early point in time in respect to onset of symptoms (e.g. chest pain) or suspicion for AMI.

The term "level", as used herein, refers to the expression level of (a) miRNA(s), which corresponds to a qualitative or quantitative assessment of the amount, number or concentration of said miRNA(s) in said blood cell sample, also referred to as miRNA profiling. Preferably, the expression level of (a) miRNA(s) is determined in a quantitative fashion. The determination of the level may be carried out by convenient means for determining the expression level of (a) miRNA(s) in said blood cell sample. The means for determining the level of (a) microRNA(s) include methods well known to the person skilled in the art, including techniques based on hybridization, amplification, enzymatic elongation or ligation, sequencing, mass spectroscopy, immune assays, flow cytometer or any combination thereof. Not limiting examples include microarray (Agilent, LC Sciences, Affymetrix, febit), next generation sequencing (ABI Solid, Illumina, Oxford Nanopores, Pacific Biosystems, Roche 454, Ion Torrent), qRT-PCR (ABI Taqman, Qiagen miScript), PCR, color-coded bead assays (Luminex), ligation-based assays (Nanostring, Firefly Bioworks), elongation-based assays (febit MPEA).

The term "reference level", refers to the expression level of (a) miRNA(s) determined in (a) control subject(s), hence (a) subject(s) not suffering from AMI. The reference level may be determined from a number of blood cell samples in a number of (control) subjects, preferably human subjects, not suffering from AMI. The reference level may be an average of a number of expression level of (a) miRNA(s) determined in number of subject not suffering from AMI.
The term "decrease" of the level of a miRNA refers to a reduced amount of miRNAs detected in the test sample in comparison to the reference level.

The level of at least one miRNA selected from the group consisting of SEQ ID NO: 1 to SEQ ID NO: 9, may be determined in a subject showing symptoms of chest pain or suspicion for a cardiovascular disease, is compared to the reference level of said at least one miRNA selected from the group consisting of SEQ ID NO: 1 to SEQ ID NO: 9, wherein a decrease of at least one miRNA selected from the group consisting of SEQ ID NO: 1 to SEQ ID NO: 9 allows for early diagnosis of AMI.

It is preferred that the reference level is determined from one or more subjects not suffering from AMI. Preferably, these subjects may be healthy controls, more preferably subjects without acute coronary syndrome, most preferably subjects without AMI.

In an alternative embodiment, the reference level is determined from one or more subjects not suffering from AMI, but suffering from other cardiovascular diseases that give rise to cardiac Troponin levels due to damage of the heart muscle. Including, but no limiting, the reference level may be determined from one or more subjects suffering from heart failure, myocarditis, pulmonary embolism.

It is alternatively of additionally preferred that the expression level in the test sample is determined within 4 hours after onset of symptoms of chest pain or other symptoms pointing to AMI. For example, the expression level in the test sample is determined within 0.1, 0.2, 0.3, 0.4, 0.5, 0.6, 0.7, 0.8, 0.9, 1.0, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9 or 4 hours after onset of symptoms of chest pain or or other symptoms pointing to AMI.

Further, it is alternatively of additionally preferred that the expression level in the test sample is determined within 2 hours after onset of symptoms of chest pain or other symptoms pointing to AMI. For example, the expression level in the test sample is determined within 0.1, 0.2, 0.3, 0.4, 0.5,0.6,0.7,0.8,0.9, 1.0, 1.2, 1.3, 1.4, 1.5, 1.6, 1.7, 1.8, 1.9 or 2 hours after onset of symptoms of chest pain or other symptoms pointing to AMI.

It is alternatively or additionally preferred, that the expression level in the test sample is determined before the level of cardiac Troponin has reached a level being above the 99^{th} percentile value of a reference population. This relates to measuring the cardiac level of Troponin (e.g. Troponin T, Troponin I) by any means in a blood sample of a subject before the concentration of cardiac Troponin has reached a level being above the 99^{th} percentile value of a reference population. For example, the level of at least one microRNA, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in a blood cell test sample from a subject is determined before the level of cardiac Troponin in that subject has reached a level being above the 99^{th} percentile value of a reference population. In a further example, the level of at least 2 microRNAs, selected from one or more sets listed in Figure 7 in a blood cell test sample from a subject is determined before the level of cardiac Troponin in that subject has reached a level being above the 99^{th} percentile value of a reference population.

It is alternatively or additionally preferred, that the expression level in the test sample is determined before the level of cardiac Troponin has reached a level of 50 pg/ml. This relates to measuring the cardiac level of Troponin (e.g. Troponin T, Troponin I) by any means in a blood sample of a subject before the concentration of cardiac Troponin reaches a value of 50pg/ml. For example, the level of at least one microRNA, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in a blood cell test sample from a subject is determined before the level of cardiac Troponin in that subject has reached a level of 50 pg/ml. In a further example, the level of at least 2 microRNAs, selected from one or more sets listed in Figure 7 in a blood cell test sample from a subject is determined before the level of cardiac Troponin in that subject has reached a level of 50 pg/ml

It is alternatively or additionally preferred, that the expression level in the test sample is determined before the level of cardiac Troponin has reached a level of 14 pg/ml. This relates to measuring the cardiac level of Troponin (e.g. Troponin T, Troponin I) by any means in a blood sample of a subject before the concentration of cardiac Troponin reaches a value of 14 pg/ml. For example, the level of at least one microRNA, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in a blood cell test sample from a subject is determined before the level of cardiac Troponin in that subject has reached a level of 14 pg/ml. In a further example, the level of at least 2 microRNAs, selected from one or more sets listed in Figure 7 in a blood cell test sample from a subject is determined before the level of cardiac Troponin in that subject has reached a level of 14 pg/ml

It is alternatively or additionally preferred, that the level of the at least one miRNA is down-regulated by a factor of at least 1.3. For example, the level determined of at least one microRNA, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in a blood cell test sample from a subject, is decreased by a factor of at least 1.3, e.g. at least by a factor of 1.3, 1.4, 1.5. ,1.6, 1.7, 1.8, 1.9, 2.0, 2.1., 2.2, 2.3, 2.4, 2.5, 2.6, 2.7, 2.8, 2.9, 3.0, 3.5, 4.0, 4.5, 5 or more, when compared to the reference level of at least one microRNA, selected from the group consisting of SEQ ID NO: 1 to SEQ ID NO: 9, preferably consisting of SEQ ID NO: 1 to SEQ ID NO: 7, more preferably consisting of SEQ ID NO: 1 to SEQ ID NO:3. In a further example, the level determined of at least 2 microRNAs, selected from one or more sets listed in Figure 7 in a blood cell test sample from a subject is decreased by a factor of at least 1.3.

It is alternatively or additionally preferred, that the level of the at least one miRNA is down-regulated by a factor of at least 1.5. For example, the level determined of at least one microRNA, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in a blood cell test sample from a subject, is decreased by a factor of at least 1.5, e.g. at least by a factor of 1.5., 1.6, 1.7, 1.8, 1.9, 2.0, 2.1., 2.2, 2.3, 2.4, 2.5, 2.6, 2.7, 2.8, 2.9, 3.0, 3.5, 4.0, 4.5, 5 or more, when compared to the reference level of at least one microRNA, selected from the group consisting of SEQ ID NO: 1 to SEQ ID NO: 9, preferably consisting of SEQ ID NO: 1 to SEQ ID NO: 7, more preferably consisting of SEQ ID NO: 1 to SEQ ID NO:3. In a further example, the level determined of at least 2 microRNAs, selected from one or more sets listed in Figure 7 in a blood cell test sample from a subject is decreased by a factor of at least 1.5.

It is alternatively or additionally preferred, that the electrocardiogram of the subject shows a ST-elevation. For example, the level of at least one microRNA, selected from the group consisting of SEQ ID NO: 1 to SEQ ID NO: 9, preferably consisting of SEQ ID NO: 1 to SEQ ID NO: 7, more preferably consisting of SEQ ID NO: 1 to SEQ ID NO:3 in a blood cell test sample from a subject is determined from subjects showing a ST-elevation in the electrocardiogram. In a further example, the level of at least 2 microRNAs, selected from one or more sets listed in Figure 7 is determined from subjects showing a ST-elevation in the electrocardiogram.

It is alternatively or additionally preferred, that at least 2 miRNAs are selected from one or more sets listed in Figure 7. For example, the level of at least 2 microRNA, selected from the group consisting of SEQ ID NO: 1 to SEQ ID NO: 9 is determined in a blood cell test sample from a subject, wherein the 2 miRNAs are selected from one or more sets of miRNAs listed in Figure 7.

Described herein is a set comprising at least one polynucleotide for detecting a set comprising at least one miRNA for early diagnosis of acute myocardial infarction in a blood cell test sample from a subject, particularly a human subject, wherein the nucleotide sequences of said at least one miRNA, e.g. 1, 2, 3, 4, 5, 6, 7, 8 or 9 miRNA(s), is selected from the group consisting of SEQ ID NO: 1 to SEQ ID NO: 9, a fragment thereof, and a sequence having at least 90% sequence identity thereto.

As to definitions of the terms "blood cell test sample", "early diagnosis", "subject", "level", "reference level" and to the preferred embodiments of "blood cell test sample", "early diagnosis", "subject", "level", "reference level" it is referred to the above explanations.

Preferably, the set comprising of at least one polynucleotide is used for detecting at least one miRNA for early diagnosis of acute myocardial infarction in a blood cell test sample from a subject, wherein the nucleotide sequence of the at least one miRNA is selected from the group consisting of SEQ ID NO: 1 to SEQ ID NO: 9, a fragment thereof, and a sequence having at least 90% sequence identity thereto. Particularly preferred, the set comprising of at least one polynucleotide is used for detecting at least one miRNA for early diagnosis of acute myocardial infarction in a blood cell test sample from a subject, wherein the nucleotide sequence of the at least one miRNA is selected from the group consisting of SEQ ID NO: 1 to SEQ ID NO: 7, a fragment thereof, and a sequence having at least 90% sequence identity thereto. More particularly preferred, the set comprising of at least one polynucleotide is used for detecting at least one miRNA for early diagnosis of acute myocardial infarction in a blood cell test sample from a subject, wherein the nucleotide sequence of the at least one miRNA is selected from the group consisting of SEQ ID NO: 1 to SEQ ID NO: 3, a fragment thereof, and a sequence having at least 90% sequence identity thereto.

It is preferred that the set comprising polynucleotides is used for detecting miRNAs for early diagnosis of acute myocardial infarction in a blood cell test sample from a subject with SEQ ID NO: 1, SEQ ID NO: 2, SEQ ID NO: 3, SEQ ID NO: 4, SEQ ID NO: 5, SEQ ID NO: 6 or SEQ ID NO: 7, a fragment thereof, or a sequence have at least 90% sequence identity thereto is determined. It is particularly preferred that the level of miRNA for early diagnosis of acute myocardial infarction in a blood cell test sample from a subject with SEQ ID NO: 1, SEQ ID NO: 2, or SEQ ID NO: 3, a fragment thereof, or a sequence have at least 90% sequence identity thereto is determined.

It is further preferred that the set of polynucleotides are used for detecting miRNAs for early diagnosis of acute myocardial infarction in a blood cell test sample from a subject with SEQ ID NO: 1, SEQ ID NO: 2, SEQ ID NO: 3, SEQ ID NO: 4, SEQ ID NO: 5, SEQ ID NO: 6 or SEQ ID NO: 7, fragments thereof, or sequences have at least 90% sequence identity thereto are determined. It is particularly preferred that the level of miRNAs for early diagnosis of acute myocardial infarction in a blood cell test sample from a subject with SEQ ID NO: 1, SEQ ID NO: 2, or SEQ ID NO: 3, fragments thereof, or a sequence have at least 90% sequence identity thereto are determined.

It is more preferred that the set comprises at least 2 polynucleotides for detection of at least 2 miRNAs for early diagnosis of acute myocardial infarction in a blood cell test sample from a subject with SEQ ID NO: 1, SEQ ID NO: 2, SEQ ID NO: 3, SEQ ID NO: 4, SEQ ID NO: 5, SEQ ID NO: 6 or SEQ ID NO: 7, fragments thereof, or sequences have at least 90% sequence identity thereto are determined. Thus, it is preferred that the set comprising 2 polynucleotides is used for detecting 2 miRNAs for early diagnosis of acute myocardial infarction in a blood cell test sample from a subject having (i) SEQ ID NO: 1 and SEQ ID NO: 2, fragments thereof, or sequences have at least 90% sequence identity thereto, (ii) SEQ ID NO: 1 and SEQ ID NO: 3, fragments thereof, or sequences have at least 90% sequence identity thereto, (iii) SEQ ID NO: 1 and SEQ ID NO: 4, fragments thereof, or sequences have at least 90% sequence identity thereto, (iv) SEQ ID NO: 2 and SEQ ID NO: 3, fragments thereof, or sequences have at least 90% sequence identity thereto, (v) SEQ ID NO: 2 and SEQ ID NO : 4, fragments thereof, or sequences have at least 90% sequence identity thereto, (vi) SEQ ID NO: 3 and SEQ ID NO: 4, fragments thereof, or sequences have at least 90% sequence identity thereto are determined.
It is further preferred that the set comprising at least 3 polynucleotides is used for detecting at least 3 miRNAs for early diagnosis of acute myocardial infarction in a blood cell test sample from a subject, having (i) SEQ ID NO 1, SEQ ID NO 2 and SEQ ID NO: 3, fragments thereof, or sequences have at least 90% sequence identity thereto, (ii) SEQ ID NO: 1, SEQ ID NO: 2 and SEQ ID NO:4, fragments thereof, or sequences have at least 90% sequence identity thereto, (iii) SEQ ID NO 1, SEQ ID NO 3 and SEQ ID NO: 4, fragments thereof, or sequences have at least 90% sequence identity thereto, (iv) SEQ ID NO: 2, SEQ ID NO: 3 and SEQ ID NO:4, fragments thereof, or sequences have at least 90% sequence identity thereto are determined.
It is further preferred, that the set comprising at least 2 polynucleotides are used for detecting at least 2 miRNAs for early diagnosis of acute myocardial infarction in a blood cell test sample from a subject, listed in Figure 7, fragments thereof, or sequences have at least 90% sequence identity thereto, are determined.

The polynucleotide, polynucleotides or set of polynucleotides or fragments thereof, are used to qualitative or quantitative determine the amount, number or concentration of said miRNA(s) for early diagnosis of acute myocardial infarction in a blood cell test sample from a subject, selected from the group consisting of SEQ ID NO: 1 to SEQ ID NO: 9, preferably SEQ ID NO: 1 to SEQ ID NO: 7, more preferably SEQ ID NO: 1 to SEQ ID NO: 3, in said blood cell test sample. Preferably, the polynucleotide, polynucleotides or set of polynucleotides are used to determine the expression level of (a) miRNA(s) in a quantitative fashion. The determination of the level may be carried out by convenient means for determining the expression level of (a) miRNA(s) in said blood cell sample by using the polynucleotide, polynucleotides or set of polynucleotides or fragments thereof. The means for determining the level of (a) microRNA(s) by use of a polynucleotide, polynucleotides or set of polynucleotides or fragments thereof, include methods well known to the person skilled in the art, including techniques based on hybridization, amplification, enzymatic elongation of ligation, sequencing, mass spectroscopy, immune assays, flow cytometer or any combination thereof. Not limiting examples include microarray (Agilent, LC Sciences, Affymetrix, febit), next generation sequencing (ABI Solid, Illumina, Oxford Nanopores, Pacific Biosystems, Roche 454, Ion Torrent), qRT-PCR (ABI Taqman, Qiagen miScript), PCR, color-coded bead assays (Luminex), ligation-based assays (Nanostring, Firefly Bioworks), elongation-based assays (febit MPEA). The polynucleotide, polynucleotides or set of polynucleotides or fragments thereof, may be attached to a solid support (e.g. beads, microarray) or present in solution (amplification, PCR, flow cytometer) or a combination thereof.

It is preferred that the set comprising polynucleotides comprises at least 2 polynucleotides for detecting a set comprising at least 2 miRNAs, for early diagnosis of acute myocardial infarction in a blood cell test sample from a subject, particularly a human subject, wherein the nucleotide sequences of said at least 2 miRNA, e.g. 2, 3, 4, 5, 6, 7, 8 or 9 miRNA(s), is selected from the group consisting of SEQ ID NO: 1 to SEQ ID NO: 9, a fragment thereof, and a sequence having at least 90% sequence identity thereto. Preferably, the set of at least 2 polynucleotides is used for detecting at least 2 miRNAs, wherein the nucleotide sequence of the at least 2 miRNAs for early diagnosis of acute myocardial infarction in a blood cell test sample from a subject is selected from the group consisting of SEQ ID NO: 1 to SEQ ID NO: 7, a fragment thereof, and a sequence having at least 90% sequence identity thereto. Particularly preferred, the set of at least 2 polynucleotides is used for detecting at least 2 miRNAs for early diagnosis of acute myocardial infarction in a blood cell test sample from a subject, wherein the nucleotide sequence of the at least 2 miRNAs is selected from the group consisting of SEQ ID NO: 1 to SEQ ID NO: 3, a fragment thereof, and a sequence having at least 90% sequence identity thereto.

It is preferred, that the set of at least 2 polynucleotides for detecting at least 2 miRNAs for early diagnosis of acute myocardial infarction in a blood cell test sample from a subject is selected from one or more sets of miRNAs listed in Figure 7.

The polynucleotide, polynucleotides or set of polynucleotides described herein may be used in the method of early diagnosis of AMI described above, may be used in a method of differentially diagnosis of AMI according to the first aspect of the present invention described below and may be used in the characterization of subjects when cardiac Troponin is used in early diagnosis of AMI in subjects with chest pain according to the second aspect of the invention described below.

In a first aspect, the invention provides a method for differential diagnosis of acute myocardial infarction, comprising the steps:
(a) determining the level of at least one miRNA with SEQ ID NO: 5 (hsa-miR-380*) in a blood cell test sample from a subject, particularly a human subject,
(b) comparing the level of said at least one miRNA of (a) with a reference level of said at least one miRNA,
(c) determining the level of cardiac Troponin in a serum or plasma test sample from said subject, and
(d) comparing the level of cardiac Troponin of (c) in with a reference level of cardiac Troponin,
   wherein the comparison of step (b) together with the comparison of step (d) allows for differential diagnosis of acute myocardial infarction and diseases that give rise to cardiac troponin levels and that are not acute myocardial infarction,
   wherein the cardiac troponin level being above the 99th percentile value of a reference population and the level of said at least one miRNA being not decreased by a factor of at least 1.5 results in the diagnosis of diseases that give rise to cardiac troponin levels and that are not acute myocardial infarction,
   wherein the cardiac troponin level being above the 99th percentile value of a reference population and the level of said at least one miRNA being decreased by a factor of at least 1.3 results in the diagnosis of acute myocardial infarction, or wherein the cardiac troponin level being not above the 99th percentile value of a reference population and the level of said at least one miRNA being decreased by a factor of at least 1.3 results in the diagnosis of acute myocardial infarction.

As to definitions of the terms "blood cell test sample", "early diagnosis", "subject", "level", "reference level" and to the preferred embodiments of "blood cell test sample", "early diagnosis", "subject", "level", "reference level" it is referred to the explanations above.

Preferably, the further miRNAs are selected from the group consisting of SEQ ID NO: 2 (hsa-miR-566), 3 (hsa-miR-455-3p) or 7 (hsa-miR-1291).

It is more preferred that the levels of at least 2 miRNAs having (i) SEQ ID NO: 2 and SEQ ID NO: 5, (ii) SEQ ID NO: 3 and SEQ ID NO: 5, and (iii) SEQ ID NO: 5 and SEQ ID NO: 7 are determined.

It is further preferred that the level of at least 3 miRNAs having (i) SEQ ID NO: 2, SEQ ID NO: 3 and SEQ ID NO: 5, (ii) SEQ ID NO: 3, SEQ ID NO: 5 and SEQ ID NO: 7, and (iii) SEQ ID NO: 2, SEQ ID NO: 5 and SEQ ID NO: 7 are determined.

It is further preferred, that the levels of a set of at least 2 miRNAs, listed in Figure 7 are determined. Preferably the set of at least 2 miRNAs is selected from SMI-3, SMI-5, SMI-11, SMI-25, SMI-29, SMI-35, SMI-48, SMI-50, SMI-51, SMI-61.

The term "differential diagnosis of acute myocardial infarction (AMI)" as referred herein, relates to a diagnostic method used to identify the presence or exclusion of AMI in a subject, where multiple alternatives are possible. These alternatives (that are not AMI) are disease that are characterized by damage to the heart muscle, resulting in release of cardiac Troponins into the circulation; these include, but are not limited to myocarditis, pulmonary embolism, heart failure, (myo)pericarditis, cardiomyopathy, (left) ventricular hypertrophy, peripartum cardiomyopathy, Takotsubo cardiomyopathy, cardiac amyloidosis, non-ischemic (systolic) heart failure.

The term "level of cardiac Troponin" relates subtypes of Troponin (e.g. cardiac Troponin I and T) that are very sensitive and specific indicators of damage to the heart muscle (myocardium), which are measured in the blood (e.g in serum or plasma) in patients e.g. with chest pain or symptoms of acute coronary syndrome.

The term "reference level of cardiac Troponin" relates to reference levels of cardiac Troponin that are determined in a blood sample in a reference population. The reference levels of cardiac Troponin I and Troponin T are determined by a blood test in a reference population. Preferably, the reference level of cardiac Troponin is above the 99^{th} percentile value of the population, more preferably, the reference value of cardiac Troponin is above 50 pg/ml, most preferably, the reference value of cardiac Troponin is above 14 pg/ml. In a preferred embodiment, the reference level of cardiac Troponin T is above the 99^{th} percentile value of the population, more preferably, the reference value of cardiac Troponin T is above 50 pg/ml, most preferably, the reference value of cardiac Troponin T is above 14 pg/ml.

It is additionally or alternatively preferred that the reference level of said at least one miRNA having SEQ ID NO: 5 (hsa-miR-380*) of step (b) in the method of differential diagnosis of AMI is determined from subjects not suffering from acute myocardial infarction. Preferably, these subjects may be healthy controls, more preferably subjects without acute coronary syndrome, most preferably subjects without AMI.

In an alternative embodiment, the reference level is determined from one or more subjects not suffering from AMI, but suffering from other cardiovascular diseases that give rise to cardiac Troponin levels due to damage of the heart muscle. Including, but no limiting, the reference level may be determined from one or more subjects suffering from myocarditis, pulmonary embolism, heart failure, (myo)pericarditis, cardiomyopathy, (left) ventricular hypertrophy, peripartum cardiomyopathy, Takotsubo cardiomyopathy, cardiac amyloidosis, non-ischemic (systolic) heart failure.

It is preferred that method of differential diagnosis allows for exclusion of diseases that give rise to a cardiac Troponin level which are not acute myocardial infarction. Including, but no limiting, these diseases comprise myocarditis, pulmonary embolism, heart failure, (myo)pericarditis, cardiomyopathy, (left) ventricular hypertrophy, peripartum cardiomyopathy, Takotsubo cardiomyopathy, cardiac amyloidosis, non-ischemic (systolic) heart failure.
It is further preferred, that the method of differential diagnosis allows for exclusion of heart failure, myocarditis or pulmonary embolism.

Preferably the method for differential diagnosis of acute myocardial infarction, comprises the steps:
(a) determining the level of at least one miRNA with SEQ ID NO: 5 (hsa-miR-380*) in a blood cell test sample from a subject, particularly a human subject,
(b) comparing the level of said at least one miRNAs of (a) with a reference level of said at least one miRNA,
(c) determining the level of cardiac Troponin in a serum or plasma test sample from said subject, and
(d) comparing the level of cardiac Troponin of (c) with a reference level of cardiac Troponin wherein the comparison of step (b) together with the comparison of step (d) allows for :
   (i.) diagnosis of acute myocardial infarction, wherein the cardiac Troponin level being above 14pg/ml, preferably above 50 pg/ml, and the level of at least one of said miRNAs being decreased by a factor of at least 1.3, preferably being decreased by a factor of at least 1.5.
   (ii.) diagnosis of diseases that give rise to a cardiac Troponin level which are not acute myocardial infarction, wherein cardiac Troponin level is above above 14pg/ml, preferably above 50 pg/ml, and the level of at least one of said miRNAs is not decreased by a factor of at least 1.5, preferably being not decreased by a factor of at least 1.3.
   (iii.) diagnosis of acute myocardial infarction, wherein the cardiac Troponin level is not above 50 pg/ml, preferably not above 14 pg/ml, and the level of at least one of said miRNAs is decreased by a factor of at least 1.3, preferably decreased by a factor of at least 1.5.

In a preferred embodiment, the method of differential diagnosis of AMI allows for the diagnosis of AMI, wherein the cardiac Troponin level is above the 99th percentile value of a reference population, and the level of at least one of said miRNAs is decreased by a factor of at least 1.5. Preferably, the method of differential diagnosis of AMI allows for the diagnosis of AMI, wherein the cardiac Troponin level is above 14 pg/ml, and the level of at least one of said miRNAs is decreased by a factor of at least 1.3. Further preferred, the method of differential diagnosis of AMI allows for the diagnosis of AMI, wherein the cardiac Troponin level is above 14 pg/ml, and the level of at least one of said miRNAs is decreased by a factor of at least 1.5.
Preferably, the method of differential diagnosis of AMI allows for the diagnosis of AMI, wherein the cardiac Troponin level is above 50 pg/ml, and the level of at least one of said miRNAs is decreased by a factor of at least 1.3. Further preferred, the method of differential diagnosis of AMI allows for the diagnosis of AMI, wherein the cardiac Troponin level is above 50 pg/ml, and the level of at least one of said miRNAs is decreased by a factor of at least 1.5.

Preferably, the method of differential diagnosis of AMI allows for diagnosis of heart failure, myocarditis or pulmonary embolism wherein the cardiac Troponin level is above the 99th percentile value of a reference population, preferably above 14pg/ml, more preferably above 50 pg/ml and the level of at least one of said miRNAs is not decreased by a factor of at least 1.5, preferably is not decreased by a factor of at least 1.3.

In an alternative preferred embodiment, the method of differential diagnosis of AMI allows for diagnosis of diseases, that give rise to a cardiac Troponin level, which are not acute myocardial infarction, wherein cardiac Troponin level is above the 99th percentile value of a reference population and the level of at least one of said miRNAs is not decreased by a factor of at least 1.3. Preferably, the method of differential diagnosis of AMI allows for diagnosis of diseases, that give rise to a cardiac Troponin level, which are not acute myocardial infarction, wherein cardiac Troponin level is above 14 pg/ml and the level of at least one of said miRNAs is not decreased by a factor of at least 1.5. Further preferred, the method of differential diagnosis of AMI allows for diagnosis of diseases, that give rise to a cardiac Troponin level, which are not acute myocardial infarction, wherein cardiac Troponin level is above 14 pg/ml and the level of at least one of said miRNAs is not decreased by a factor of at least 1.3.
Preferably, the method of differential diagnosis of AMI allows for diagnosis of diseases, that give rise to a cardiac Troponin level, which are not acute myocardial infarction, wherein cardiac Troponin level is above 50 pg/ml and the level of at least one of said miRNAs is not decreased by a factor of at least 1.5. Further preferred, the method of differential diagnosis of AMI allows for diagnosis of diseases, that give rise to a cardiac Troponin level, which are not acute myocardial infarction, wherein cardiac Troponin level is above 50 pg/ml and the level of at least one of said miRNAs is not decreased by a factor of at least 1.3.

Further preferred in the method of differential diagnosis of AMI that allows for diagnosis of diseases, that give rise to a cardiac Troponin level, which are not acute myocardial infarction, the set comprising miRNAs in step (a) is selected from the group consisting of (i) SEQ ID NO: 2 and SEQ ID NO: 5, (ii) SEQ ID NO: 3 and SEQ ID NO: 5, (iii) SEQ ID NO: 5 and SEQ ID NO: 7, (iv) SEQ ID NO: 2, SEQ ID NO: 3 and SEQ ID NO: 5, (v) SEQ ID NO: 3, SEQ ID NO: 5 and SEQ ID NO: 7, (vi) SEQ ID NO: 2, SEQ ID NO: 5 and SEQ ID NO: 7.

In an additional preferred embodiment, the method of differential diagnosis of AMI allows for the diagnosis of AMI, wherein the cardiac Troponin level is not above the 99th percentile value of a reference population, and the level of at least one of said miRNAs is decreased by a factor of at least 1.5.
Preferably, the method of differential diagnosis of AMI allows for the diagnosis of AMI, wherein the cardiac Troponin level is not above 14 pg/ml, and the level of at least one of said miRNAs is decreased by a factor of at least 1.3. Further preferred, the method of differential diagnosis of AMI allows for the diagnosis of AMI, wherein the cardiac Troponin level is not above 14 pg/ml, and the level of at least one of said miRNAs is decreased by a factor of at least 1.5. Preferably, the method of differential diagnosis of AMI allows for the diagnosis of AMI, wherein the cardiac Troponin level is not above 50 pg/ml, and the level of at least one of said miRNAs is decreased by a factor of at least 1.3. Further preferred, the method of differential diagnosis of AMI allows for the diagnosis of AMI, wherein the cardiac Troponin level is not above 50 pg/ml, and the level of at least one of said miRNAs is decreased by a factor of at least 1.5.

It is preferred that in the method of differential diagnosis at the level of least 2 miRNAs in a blood cell sample of a subject are determined from one or more set(s)of miRNA(s) are selected from the list in Figure 7.

In a second aspect, the invention provides the use of cardiac Troponin in early diagnosis of acute myocardial infarction in subjects, preferably human subjects, with chest pain characterized by a cardiac troponin level being above the 99th percentile value of a reference population, preferably above 14pg/ml, more preferably above 50 pg/ml and by a not decreased level of a factor of at least 1.5, preferably by a not decreased level of a factor of at least 1.3 of at least one miRNA in a blood cell test sample from said subject, wherein said at least one miRNA is selected from SEQ ID NO: 5 (hsa-miR-380*).

As to definitions of the terms "blood cell test sample", "early diagnosis", "subject", "level", "reference level" and to the preferred embodiments of "blood cell test sample", "early diagnosis", "subject", "level", "reference level" it is referred to the first aspect of the present invention.

It is further preferred, that the subjects are characterized by a not decreased level of a set of miRNAs, listed in Figure 7. It is further particularly preferred, that the subjects are characterized by a not decreased level of miRNAs selected from one or more sets listed in Figure 7.

More preferably, the subjects are characterized by a set of at least 2 miRNAs selected from one or more sets listed in Figure 7.

Described herein is a kit for early diagnosis and/or differential diagnosis of acute myocardial infarction in test sample from a subject comprising means for determining the expression level of at least one miRNA in a blood cell test sample and optional means for determining the level of cardiac troponin in a serum or plasma test sample of the subject.

As to definitions of the terms "blood cell test sample", "early diagnosis", "subject", "level", "reference level" and to the preferred embodiments of "blood cell test sample", "early diagnosis", "subject", "level", "reference level" it is referred to the above explanations.

The means for determining the expression level of at least one miRNA in a blood cell test sample of a subject, include- but are not limited to - technologies employing one or more methods selected from nucleic acid amplification (e.g. PCR, RT-PCR, LCR, rolling circle amplification), nucleic acid hybridization (e.g. microarrays, branched-dna, beads, microspheres), nucleic acid sequencing (e.g. Sanger-sequencing, next-generation-sequencing, pyro-sequencing, sequencing-by-hybridisation, sequencing-by-synthesis) or flow cytometry. It is preferred to employ RT-PCR, microarrays or next-generation-sequencing for determination of the expression level of at least one miRNA selected from the group consisting of SEQ ID NO: 1 to SEQ ID NO: 9, preferably selected from the group consisting of SEQ ID NO: 1, SEQ ID NO: 2, SEQ ID NO: 3, SEQ ID NO: 4, SEQ ID NO: 5, SEQ ID NO: 6 or SEQ ID NO: 7, for early diagnosis and/or differential diagnosis of acute myocardial infarction in a blood cell sample from a subject

It is preferred that the nucleotide sequence of the at least one miRNA to be determined is selected from the group consisting of SEQ ID NO: 1 to SEQ ID NO: 9, preferably selected from the group consisting of SEQ ID NO: 1, SEQ ID NO: 2, SEQ ID NO: 3, SEQ ID NO: 4, SEQ ID NO: 5, SEQ ID NO: 6 or SEQ ID NO: 7, for early diagnosis and/or differential diagnosis of acute myocardial infarction in a blood cell sample from a subject. It is particularly preferred that the level of at least one miRNA with SEQ ID NO: 1, SEQ ID NO: 2, or SEQ ID NO: 3 is determined, in particular for early diagnosis of acute myocardial infarction. It is further particularly preferred that the level of at least one miRNA with SEQ ID NO: 2, SEQ ID NO: 3, SEQ ID NO: 5 or SEQ ID NO: 7 is determined, in particular for differential diagnosis of acute myocardial infarction.
The means for determining the level of cardiac troponin in a serum or plasma test sample of a subject include -but are not limited to - immunoassay-based blood tests, e.g. manufactured by Roche, Siemens, Abbott and others.
It is preferred that the kit for early diagnosis of and/or differential diagnosis of acute myocardial infarction in a blood cell sample from a subject, wherein the nucleotide sequence of the at least one miRNA is selected from the group consisting of SEQ ID NO: 1 to SEQ ID NO: 7.

It is preferred that the kit further comprises :
(i) means for collecting the blood test sample(s) and/or
(ii) means for stabilizing the RNA-fraction in the blood cell test sample
(iii) a data carrier

Said kit may further comprise means for collecting the blood test sample and/or means for stabilizing the RNA-fraction, especially the small RNA-fraction. Preferably, the means for collecting the test sample may be a test sample collection container, more preferably a blood collection tube, most preferably a blood collection tube suitable for analysing the RNA-fraction (including miRNAs) or the protein fraction (including cardiac troponins). Preferably, the means for stabilizing the RNA fraction, especially the small RNA-fraction may either added to the test sample during or after blood collection (e.g. by adding RNAlater, RNAretain) or are already included in the blood collection tube (e.g PAXgene tube, PAXgene blood RNA tube, Tempus blood RNA tube). Most preferably, the means for collecting the test sample and the means for stabilizing the RNA-fraction, especially the small RNA-fraction, are comprised in a blood collection tube that contains means for stabilizing the said RNA-fraction, especially said small RNA-fraction (e.g. PAXgene blood RNA tube, Tempus blood RNA tube).

Said kit may further comprise a data carrier. The data carrier may be an electronical or a non-electronical data carrier which contains information about use and/or analysis of the kit for early diagnosis and/or differential diagnosis of acute myocardial infarction. The information comprised in the kit holds instructions on how to use the kit and/or on how to analyze the data obtained after using the kit.
Steps for using the kit may include - but are not limited to - obtaining the test sample from the test subject (e.g. blood draw), preparation of the blood cell test sample from the test sample, storage of the sample or fractions thereof, work-up of the test sample or fractions thereof, shipping of the test sample or fractions thereof, isolation of the RNA from the test sample or fractions thereof, determination of the expression level of at least one miRNA selected from the group consisting of SEQ ID NO: 1 to SEQ ID NO: 9, preferably selected from the group consisting of SEQ ID NO: 1 to SEQ ID NO: 7, determination of the cardiac troponin (e.g. troponin I, troponin T) levels from the plasma or serum test samples.
Steps for analyzing the data may include - but are not limited to - read-out of the data (miRNA expression level, troponin level) after determining the expression or troponin level (see above), storing the data, background substraction, normalization of the data, statistical analysis of the data, determining a test score from the test data, comparing the test data or a test score of the test sample to reference data (e.g. reference miRNA expression level, reference troponin level, reference scores), (instructions for) evaluation of the miRNA expression data in view of the troponin data (an vice versa), determining a statistical measure of a likelihood (e.g. a predictive value) of suffering or not suffering from acute myocardial infarction, determining a statistical measure of a likelihood (e.g. a predictive value) of suffering or not suffering from a disease that gives rise to cardiac troponin levels which is not acute myocardial infarction (e.g. heart failure, cardiomyopathy, myocarditis, pulmonary embolism). The aforementioned steps for analyzing the data may be partially or fully supported by mathematical algorithms or software tools.

Further described herein is the following:
1. A method for early diagnosis of acute myocardial infarction, comprising the steps :
   (a) determining the level of at least one microRNA selected from the group consisting of SEQ ID NO: 1 to SEQ ID NO: 9, a fragment thereof, or a sequence have at least 90% sequence identity thereto, in a blood cell test sample from a subject, particularly a human subject,
   (b) comparing the level of said miRNA of step (a) with the reference level of said at least one miRNA
      wherein a decrease of the level of said at least one miRNA in the test sample in comparison to the reference level allows for early diagnosis of acute myocardial infarction
2. The method of item 1, wherein the reference level is determined from subjects not suffering from acute myocardial infarction
3. The method of any of the items 1 to 2, wherein the expression level in the test sample is determined within 4 hours after onset of symptoms of chest pain
4. The method of 1 to 3, wherein the expression level in the test sample is determined within 2 hours after onset of symptoms of chest pain
5. The method of any of the items 1 to 4, wherein the expression level in the test sample is determined before the level of cardiac Troponin has reached a level of 50 pg/ml
6. The method of any of the items 1 to 5, wherein the expression level in the test sample is determined before the level of cardiac Troponin has reached a level of 14 pg/ml
7. The method of any of the items 1 to 6, wherein the level of the at least one miRNA is down-regulated by a factor of at least 1.3
8. The method of any of the items 1 to 7, wherein the level of the at least one miRNA is down-regulated by a factor of at least 1.5
9. The method of any of the items 1 to 8, wherein the electrocardiogram of the subject shows a ST-elevation
10. The method of any of the items 1 to 8, wherein the electrocardiogram of the subject does not show a ST-elevation
11. The method of any of the items 1 to 10, wherein at least 2 miRNAs are selected from one or more sets listed in Figure 7
12. A set comprising at least one polynucleotide for detecting a set comprising at least one miRNA for early diagnosis of acute myocardial infarction in a blood cell test sample from a subject, particularly a human subject, wherein the nucleotide sequences of said at least one miRNA is selected from the group consisting of SEQ ID NO: 1 to SEQ ID NO: 9, a fragment thereof, and a sequence having at least 90% sequence identity thereto.
13. The set of polynucleotides of item 12, wherein the set comprises at least 2 miRNAs
14. The set of polynucleotides of item 13, wherein the set is selected from one or more sets of miRNAs listed in Figure 7
15. A method for differential diagnosis of acute myocardial infarction, comprising the steps :
   (a) determining the level of at least one microRNA selected from the group consisting of SEQ ID NO: 1 to SEQ ID NO: 9, a fragment thereof, or a sequence having at least 90% sequence identity thereto, in a blood cell test sample from said subject, particularly a human subject
   (b) comparing the level of said miRNAs of (a) with the reference level of said at least one miRNA
   (c) determining the level of cardiac Troponin in a serum or plasma test sample from said subject,
   (d) comparing the level of cardiac Troponin of (c) in with the reference level of cardiac Troponin
      wherein the comparison of step (b) together with the comparison of step (d) allows for differential diagnosis of acute myocardial infarction
16. The method of item 15, wherein the reference level of (b) is determined from subjects not suffering from acute myocardial infarction
17. The method of any of the items 15 to 16, wherein the differential diagnosis allows for exclusion of diseases that give rise to a cardiac Troponin level which are not acute myocardial infarction
18. The method of items 17, wherein the differential diagnosis allows for exclusion of heart failure, myocarditis or pulmonary embolism
19. The method of any of the items 15 to 16, wherein the cardiac Troponin level is above the 99th percentile value of a reference population, preferably above 14pg/ml, more preferably above 50 pg/ml and the level of at least one of said miRNAs is decreased by a factor of at least 1.3, preferably is decreased by a factor of at least 1.5 resulting in the diagnosis of acute myocardial infarction
20. The method of any of the items 15 to 16, wherein cardiac Troponin level is above the 99th percentile value of a reference population, preferably above 14pg/ml, more preferably above 50 pg/ml and the level of at least one of said miRNAs is not decreased by a factor of at least 1.5, preferably is not decreased by a factor of at least 1.3 resulting in a diagnosis of diseases that give rise to a cardiac Troponin level which are not acute myocardial infarction
21. The method of any of the items 15 to 16, wherein the cardiac Troponin level is not above the 99th percentile value of a reference population, preferably not above 50 pg/ml, more preferably not above 14 pg/ml and the level of at least one of said miRNAs is decreased by a factor of at least 1.3, preferably decreased by a factor of at least 1.5 resulting in diagnosis of acute myocardial infarction
22. The method of item 20, wherein heart failure, myocarditis or pulmonary embolism are diagnosed
23. The method of any of the items 15 to 22, wherein at least 2 miRNAs are selected from one or more sets listed in Figure 7
24. Cardiac Troponin for use in early diagnosis of acute myocardial infarction in patients with chest pain, wherein the patients are characterized by a decreased level of at least one miRNA(s) in a blood cell test sample, selected from the group consisting of SEQ ID NO: 1 to 9, a fragment thereof, or a sequence having at least 90% sequence identity thereto, compared to a reference level
25. Cardiac Troponin for use in early diagnosis according to item 24, wherein the patients are further characterized by a cardiac Troponin level above the 99th percentile value of a reference population, preferably above 14pg/ml, more preferably above 50 pg/ml and the level of at least one of said miRNAs is decreased by a factor of at least 1.3, preferably is decreased by a factor of at least 1.5.
26. Cardiac Troponin for use in early diagnosis according to item 24, wherein the patients are further characterized by a cardiac Troponin level above the 99th percentile value of a reference population, preferably above 14pg/ml, more preferably above 50 pg/ml and the level of at least one of said miRNAs is not decreased by a factor of at least 1.5, preferably is not decreased by a factor of at least 1.3.
27. Cardiac Troponin for use in early diagnosis according to item 24, wherein the patients are further characterized by a cardiac Troponin level not above the 99th percentile value of a reference population, preferably not above 50 pg/ml, more preferably not above 14 pg/ml and the level of at least one of said miRNAs is decreased by a factor of at least 1.3, preferably is decreased by a factor of at least 1.5.
28. Cardiac Troponin for use in early diagnosis according to any of the items 24 to 27, wherein at least 2 miRNAs are selected from one or more sets listed in Figure 7
29. Use of a set of polynucleotides according to any of the items 12 to 14 for early diagnosis and/or differential diagnosis of acute myocardial infarction in a blood cell sample from a subject.
30. A kit for early diagnosis of and/or differential diagnosis of acute myocardial infarction in a test sample from a subject comprising means for determining the expression level of at least one miRNA in a blood cell test sample and optional means for determining the level of cardiac troponin in a serum or plasma test sample of the subject wherein the nucleotide sequence of the at least one miRNA is selected from the group consisting of SEQ ID NO: 1 to SEQ ID NO: 9.
31. The kit of item 30, wherein the kit further comprises :
   (i) means for collecting the blood test sample(s) and/or
   (ii) means for stabilizing the RNA-fraction in the blood cell test sample
   (iii) a data carrier
32. The kit of item 31, wherein said data carrier is an electronical or a non-electronical data carrier which contains information about use and/or analysis of the kit for early diagnosis and/or differential diagnosis of acute myocardial infarction.

### BRIEF DESCRIPTION OF THE DRAWINGS

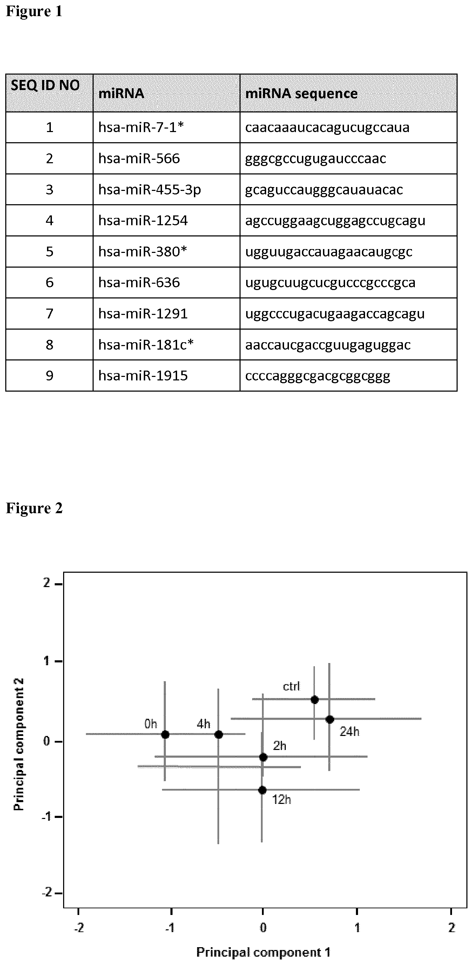

Figure 1: MiRNAs for early diagnosis and/or differential diagnosis of AMI. With SEQ ID NO: sequence identification number, miRNA: identifier of the miRNA according to miRBase, miRNA sequence : in 5'-3' orientation.
Figure 2: Changes in miRNA expression in AMI patients are most evident in the early stage of myocardial infarction. Principle component analysis visualizing miRNA expression at all measured time-points and in comparison to a control group (ctrl). MiRNA expression levels of the AMI group are most distant from healthy controls at presentation to hospital (0h).

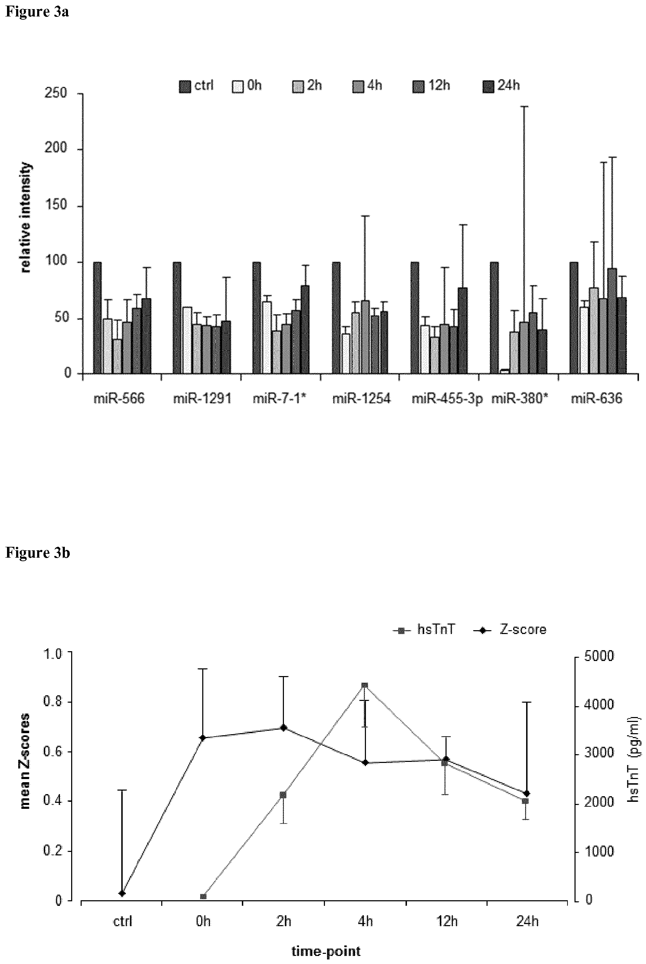

Figure 3: Set (signature) of 7 consistently dysregulated miRNAs is an early marker of AMI. a) Bar graphs showing miRNAs that are down-regulated at each time-point studied. Bars show relative intensity values of hsa-miR-566, -1291, -7-1*, -1254, -455-3p, -380* and -636 at different time-points normalized to controls (ctrl). b) miRNA dysregulation is detectable at a very early time point. Already at the presentation to hospital (0h), averaged Z-scores of the signature comprising the 7 miRNAs discriminate AMI patients that show Troponin levels <50pg/ml.

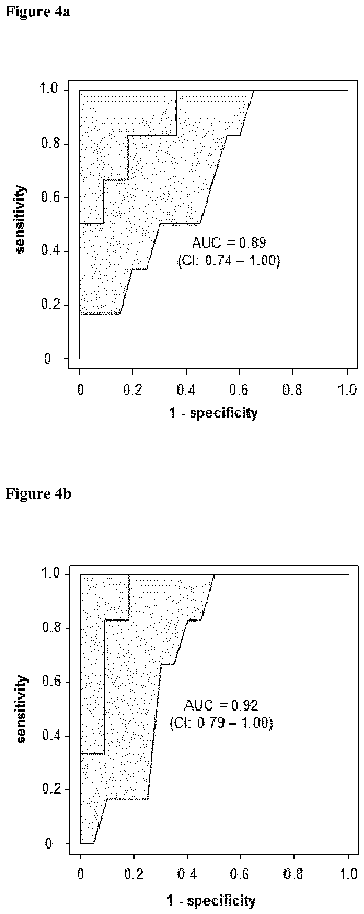

Figure 4: Predictive values of the miRNA signature at early time-points. a-b) Receiver operating characteristics (ROC) analysis of the 7 miRNA signature at presentation to the hospital (a) and two hours after admission (b). Blue shaded areas indicates the corresponding confidence intervals (CI).

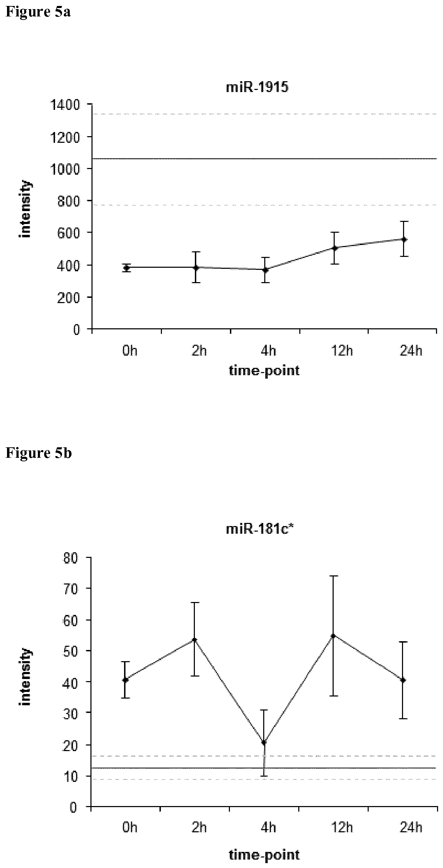

Figure 5: hsa-miR-1915 and hsa-miR-181c* are novel miRNAs associated with myocardial infarction. In AMI patients, hsa-miR-1915 is one of the most significantly downregulated miRNAs (a), while hsa-miR-181c* shows consistent upregulation with the exception of time point 3 (b). The red line indicates the mean intensity values in the control group, dashed lines indicate the corresponding SEM
Figure 6: Graphical representation of timecourse measurements of miRNAs with SEQ ID NO: 1 to SEQ ID NO: 7 in blood cell test samples of AMI-patients (STEMI-patients) for early diagnosis and/or differential diagnosis of AMI; Y-axis : relative fluorescence intensities obtained from microarray analysis; x-axis : 5 time points of measurement (0, 2, 4, 12, 24 hours after initial presentation in the hospital) and additional 2 control subject cohorts, namely healthy controls and dilated cardiomyopathy (DCM) patients.
Figure 7: List of sets (signatures) of miRNAs for early diagnosis and/or differential diagnosis of acute myocardial infarction. With signature number SMI-1 to SMI-61; SEQ ID NO: sequence identification number, miRNA: identifier of the miRNA according to miRBase.

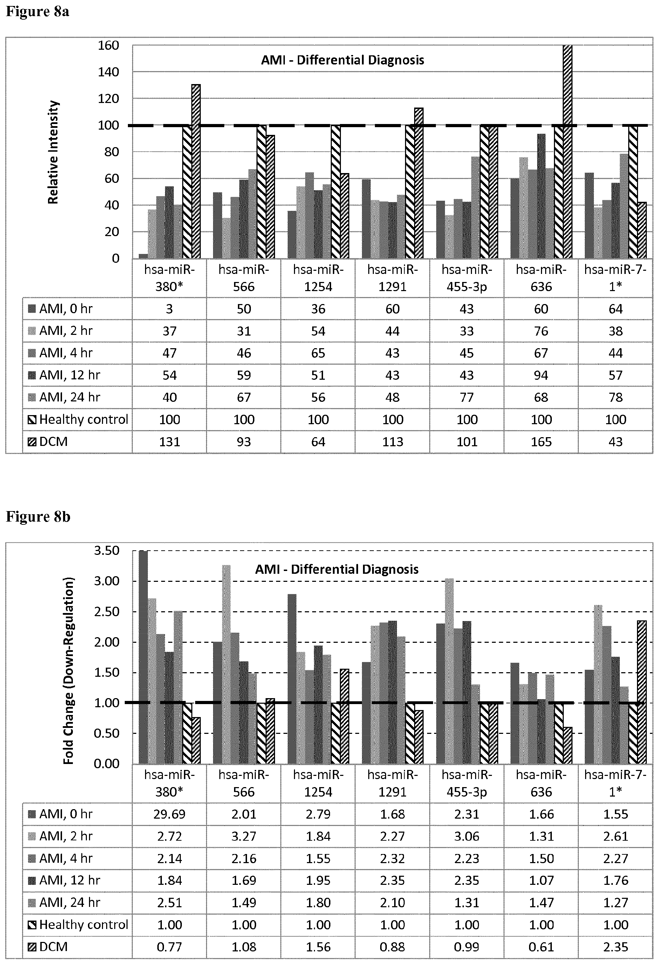

Figure 8: Differential Diagnosis of AMI. (a) Relative fluorescence intensities obtained from microarray analysis with miRNAs of SEQ ID NO: 1 to SEQ ID NO: 7 in blood cell test samples of AMI-patients (STEMI-patients at 0, 2, 4, 12, 24 hours after initial presentation in the hospital), healthy controls and dilated cardiomyopathy (DCM) patients. The relative fluorescence intensitites are normalized to healthy controls. (b) Fold Change downregulation (fold decrease of miRNA level) relative to healthy controls calculated from data of Figure 8a obtained from microarray analysis with miRNAs of SEQ ID NO: 1 to SEQ ID NO: 7 in blood cell test samples of AMI-patients (STEMI-patients at 0, 2, 4, 12, 24 hours after initial presentation in the hospital), healthy controls and dilated cardiomyopathy (DCM) patients.

### EXAMPLES

The Examples are designed in order to further illustrate the present invention and serve a better understanding. They are not to be construed as limiting the scope of the invention in any way.

Alterations in microRNA (miRNA) expression patterns are thought to be potential biomarkers for several cardiovascular disorders. We previously reported 121 microRNAs to be significantly dysregulated in acute myocardial infarction (AMI) and applied machine learning techniques to define miRNA subsets with high diagnostic power. However, the kinetics of these novel miRNA biomarkers remained elusive. To further characterize temporal changes in the human miRNome, we performed here the first whole-genome miRNA kinetic study in AMI patients by measuring miRNA expression levels at multiple time-points (0, 2, 4, 12, 24 hours after initial presentation) in patients with acute ST-Elevation Myocardial Infarction (STEMI) by using microfluidic primer extension arrays. As a prerequisite, all patients enrolled had high sensitive Troponin levels below 50 pg/ml at hospital admission. We found a subset of previously identified miRNAs to be significantly (p = 0.002) dysregulated already at initial presentation and during the course of AMI. Additionally, we could identify novel miRNAs that are associated with early-stage myocardial infarction, such as miR-1915 and miR-181c*. In summary, the present study provides the first insights into the dynamic properties of the human disease miRNome in the course of an acute cardiovascular event.

Acute myocardial infarction (AMI) caused by coronary artery occlusion leads to cardiomyocyte death and subsequent cardiac necrosis (1). In the course of this process, various molecular cascades are activated and different molecules and proteins are released from the cardiomyocyte's cytosol into the blood stream. One of these proteins is cardiac Troponin T. Due to its cardio-specificity it has become the key biomarker for detection of myocardial damage. A rise and/or fall of cardiac Troponin T or I with at least one value above the 99th percentile value of a reference population is today an integral part of the universal definition of myocardial infarction (2-5). However, Troponins do also have some inherent limitations as biomarkers for AMI (29, 30). For instance, they reflect myocardial damage only with a certain delay since they need to be released to the blood stream from undergoing cardiomyocytes. In 5th generation high sensitive Troponin assays this delay has been significantly reduced, allowing now an earlier diagnosis. However, the reduction in the detection limit goes along with a decreased clinical specificity and elevated Troponins are not necessarily due to acute coronary syndromes but can be caused by other acute or chronic alterations of the cardiovascular system, such as pulmonary embolism, myocarditis, or heart failure. Accordingly, an early and specific discrimination of acute coronary syndromes could benefit from a multiple biomarker strategy, combining the diagnostic power of different biomarkers to circumvent these limitations.
MiRNAs are small non-coding nucleotides that influence on the expression of a wide variety of target genes (6, 7). They not only regulate physiological mechanisms such as differentiation, proliferation or apoptosis, but also enable the cell to adapt to pathophysiological conditions of ischemia, hypertrophy, or arrhythmias (8-12). Hence, miRNAs might also serve as potential non-invasive biomarkers for cardiovascular disorders (13-15). In a genome-wide approach, we recently were first to dissect the alterations of the miRNome in whole peripheral blood of AMI patients (14). A total of 121 miRNAs had been found to be significantly dysregulated in AMI, with miR-1291 and miR-663b showing the highest sensitivity and specificity in discriminating AMI from control patients. We also reported subset signatures of miRNAs that predict myocardial infarction with even higher statistical power. However, the kinetics of the changes in miRNA levels remained illusive but is of great importance to qualify these miRNAs as novel biomarkers and provide insights into the dynamics of the miRNome in acute disorders.
We have assessed here serial blood samples from patients presenting with early-stage ST-Elevation Myocardial Infarction (STEMI) (hsTnT levels below 50 pg/nl on hospital admission) and analysed whole-genome miRNA expression profiles at 5 defined time points using microfluidic primer extension arrays (16). The results validate previously described AMI miRNAs as early markers and refine a signature of 7 miRNAs that is significantly dysregulated already on initial hospital admission, indicating that miRNAs may be useful novel biomarkers particularly suited for early diagnosis of AMI.

### Materials and methods

### Study Design

20 patients with suspected STEMI were recruited during the course of this study. From those, 7 patients representing 35 samples over the 5 time-points with STEMI and Troponin levels <50 pg/ml, as well as 11 controls without acute coronary syndrome were enrolled in the present study. 13 patients were excluded since their admission cTnT levels exceeded 50 pg/ml or diagnosis of AMI was rejected by invasive angiography, e.g. due to an underlying myocarditis. All patients with STEMI underwent immediate heart catheterization and PCI of the culprit lesion. The patients in the control group were investigated by routine coronary angiography for suspected coronary artery disease. They showed neither >50% coronary artery stenosis nor elevations in cTnT (see Table 1 for detailed clinical characteristics). Patients and controls had given written informed consent and the study has been approved by the local ethics committee. Serial cTnT levels were assessed using Elecsys highly sensitive Troponin T assay (hsTnT; Roche, Germany). All patients enrolled had hsTnT levels < 50pg/ml at baseline and two patients had hsTnT levels < 14pg/ml, which is the cut-off for 5. generation Troponin assays).

### MiRNA expression profiling

In order to quantify the complete miRNome we used a microfluidics microarray in combination with a primer extension assay (microfluidics primer extension assay, MPEA) (16). In brief, total RNA from peripheral whole blood collected in PAXgene tubes was extracted as described previously (17). Biochips with 7 replicates of all known miRNAs (miRbase 15) (18) were hybridized with the unlabeled and non-amplified RNA using the Geniom RT Analyzer (febit, Heidelberg). On top of each capture probe on the biochip a poly-Thymidin-tail has been synthesized. Klenow polymerase then binds to the hybridized miRNA/probe complex and extends the miRNA with biotinilated Adenins. The signals were measured by a CCD camera and signals analysed by the Geniom Wizard software (febit, Germany). All signals were background corrected and the median expression of the 7 capture probes was computed. Finally, quantile normalization (19) was applied and for all further computations, the normalized expression intensities were used.

### Principal Component Analysis

To visualize the high-dimensional distribution of all miRNAs in a two-dimensional subspace, we applied principal component analysis (PCA). In brief, an Eigenvalue-Decomposition of the miRNA expression matrix was carried out and based on this, the principal components (pc) were computed. Essentially, the first few pc's representing linear combinations of the original patients' profiles carry the largest part of the overall information. In our case we computed the first and second pc for each patient separately. Then, for each of the 5 consecutive time-points and controls the average and standard deviation of the pc's was plotted. This type of representation is well suited to indicate proximity of miRNA expression levels at the different time-points including control patients.

### Z-scores of biomarker signatures

To grasp whether a patient has a positive or a negative miRNA profile for AMI, a well interpretable statistical measure is essential. Here, classification technologies represent a common approach in the basic research field, while they are yet rarely used in clinical practice. To address this point we decided on a well interpretable measure, Z-scores. A Z-score indicates how many standard deviations a patient's biomarker value is above or below a populations average. A single threshold then can be applied to determine whether patients are positive or negative. To extend this concept to sets of miRNAs, we consider the median z-score of all signature miRNAs, representing a stable estimate of the overall degree of dysregulation.

### Results

### Study design and patient characteristics

We previously reported a total of 121 miRNAs to be significantly dysregulated in AMI patients including subsets of miRNAs that predict AMI with high statistical power (14). However, it remained unclear when exactly these changes in miRNA expression are detectable at the earliest. To address this important question, we assessed serial blood samples from 7 patients with AMI beginning at their admission to the hospital (inclusion criterion: hsTnT < 50 pg/ml), and compared them to 11 matched control patients without relevant coronary artery disease and without suspicion of ACS (Table 1). The included patients did not show any significant differences regarding their age, gender, or renal function. In the AMI group, more patients were current smokers (72% and 27%, respectively, p=0.009), but other cardiovascular risk factors were equally distributed between the two groups.

### Altered expression levels of specific miRNAs are detectable in the early course of AMI

To comprehensively analyse miRNA expression changes in patients and controls, we applied whole-genome miRNA measurements for all samples (n = 46). First, to assess how the expression patterns of miRNAs change over time, we performed a principle component analysis of the previously identified (14) and most strongly dysregulated miRNAs to visualize their distribution at the specific time-points in a two dimensional space (Fig. 2). Interestingly, miRNA expression levels in AMI patients are most distant from the controls (denoted as 'ctrl' in Fig.2) at the earliest time-point (0 h), meaning at their initial presentation to the hospital. In the course of myocardial infarction, expression differences decrease such that 24 h after presentation, miRNA levels in AMI patients are almost comparable to the control group.

### A signature consisting of 7 miRNAs as an early marker of AMI

We next evaluated if the most significantly dysregulated miRNAs are able to indicate the presence of early myocardial infarction. To this end, we first applied a filtering approach with the 40 most significantly dysregulated miRNAs (according to p-values) previously described and excluded miRNAs that are lowly abundant. Hereby, we removed 16 of the 40 miRNAs (40%) whose maximal expression was below the limit of 50 fluorescence intensity units. Of the remaining 24 markers, we compared the median fold changes between AMI patients and controls, irrespectively of the time-point. We found that 17 of the 24 miRNAs (71 %) were likewise dysregulated compared to the initial study, while the remaining 7 miRNAs (29%) showed no significant concordance. These 17 markers, of which 2 were up- and 15 were down-regulated in AMI patients, showed a high consistency between the different time-points. In detail, 5 of them (29%) showed a discrepancy in 2 of 5 time-points and further 5 showed a discrepancy in only one of 5 time-points. Most remarkably, the remaining 7 markers were dysregulated in the same direction at all time-points. These miRNAs include human miR-636, miR-7-1*, miR-380*, miR-1254, miR-455-3p, miR-566, and miR-1291 (Fig. 3 A).

Although each of these 7 miRNAs may individually represent a promising biomarker for the early detection of AMI, a combined signature could additionally increase the diagnostic power. Thus, we combined these markers and computed Z-scores for each patient at each time-point and for each miRNA. In general, a Z-score reflects how many standard deviations a marker is above or below the corresponding value in a reference group. To combine the predictive power of different markers, we summed up the Z-scores of up-regulated miRNAs and the negative values of down-regulated miRNAs. Finally, the average was calculated. Additionally, the Z-score distribution has been cut at -1.645 and +1.645, respectively, cutting 5% at the left and right part of the distribution. By applying this approach, we computed scores for the 7 markers and all patients at all time-points. As shown in Figure 3B, the average Z-scores of patients show highest values at time-point 1 and 2, corresponding to 0h and 2h after presentation to the hospital. For time-point 5, the average Z-scores start to return to almost normal values, as already found by the principal component analysis for all miRNAs (Figure 2). Considering the statistical significance, all time-points are significantly different as compared to the control cohort besides time-point 5. The respective two-tailed p-values were 0.004, 0.002, 0.013, 0.007 and 0.082, respectively. Interestingly, the two earliest time-points following presentation were the most significant ones.
Although the sample size of the study was limited and hence these analysis are not able to finally conclude on the discriminatory power, we next computed receiver operator characteristic curves (ROC) for the early AMI signature and found high AUC (area under the curve) values (20) for the first and second time-point. In detail, AUC values were 0.89 (CI: 0.74 - 1.00) and 0.92 (CI: 0.79 - 1.00), respectively, as detailed in the ROC curves in Figure 4. While for time-point 1, our approach yielded a specificity of 81.8% and sensitivity of 83.3%, for time-point 2, we were able to reach the same specificity and a sensitivity of 100%.

### Identification of novel miRNAs associated with early-stage AMI

In addition to the validation and refinement of previously identified miRNAs for the early detection of AMI, the assessment of the whole miRNome of patients and controls represents a comprehensive source to screen for novel diagnostic miRNAs. After ranking all miRNAs and time points according to p-values, we additionally find miR-1915 and miR-181c* to be significantly associated with the presence of AMI at the different stages examined (Fig. 5A and B). While miR-1915 showed downregulation at all 5 time points, miR-181c* was significantly upregulated during AMI with the exception of the 4h blood samples.

### Discussion

We recently identified miRNAs as potential novel biomarkers for AMI (14) and demonstrated their high statistical power in discriminating AMI from control patients. To now elucidate the kinetics of miRNA dysregulation, we performed here the first serial whole-miRNome expression study in AMI patients. We were able to validate previous miRNAs in an independent patient cohort and confirmed their validity during the course of myocardial infarction. In detail, we identified a signature of 7 of these miRNAs to be dysregulated at all time-points studied. MiR-1291, which was previously shown to have the highest discriminatory power as a single marker, was also amongst the top 7 miRNAs, underlining the value of miR-1291 as potential AMI biomarker (14). By now, miRNAs have been shown to be prospective biomarkers e.g. for cancers, neurological disorders, and cardiovascular disease (13). However, many studies on miRNA expression patterns in human disorders were mainly based on the evaluation of single time points, neglecting the rather dynamic nature of most diseases. Hence, the dynamics of the human miRNome in health and disease is largely unknown. Nevertheless, the detailed understanding of the temporal changes of miRNAs networks has huge implications for the development of broadly applicable diagnostic tests. Accordingly, we performed here a study unravelling the kinetics of the miRNome in patients with acute myocardial infarction. As shown, the observed changes occur very rapidly and resolve during the first 2 days after the initial event in patients after reperfusion therapy and with reestablished coronary blood flow. Surprisingly, the two earliest time-points showed the most significant dysregulation of 7 miRNAs. Notably, at time-point 1 hsTnT levels were still below the cut-off value of < 50 pg/ml (cut-off for 4. generation Troponin assays) and even below 14 pg/ml (cut-off for 5. generation Troponin assays) in 2 patients. Hence, these miRNAs are very early indicators of AMI and might be dysregulated even before molecules egress from injured cardiomyocytes. Besides the validation of previously known AMI miRNAs, evaluation of the miRNome kinetics allowed identification of additional miRNAs that are associated with early-stage AMI, with miR-1915 showing reduced expression and miR-181c* upregulation in STEMI patients.
The precise expression patterns and biological functions of the here-described miRNAs are still unknown. It is likely that the miRNAs comprising the early MI signature are not heart specific, but are derived from various cell types including inflammatory cells, activated thrombocytes, or injured endothelium. To our knowledge, none of the 7 AMI miRNAs derived from peripheral blood were associated with a certain disease. However, some of them seem to be associated with malignancies in the according tumor tissue (21). For instance, miR-455-3p seems to be associated with the acquisition of temozolomide resistance in glioblastoma multiforme (22) and miR-1915 influences on Bcl-2-mediated drug resistance in human colorectal carcinoma cells (23). Interestingly, miR-181 which we identified as novel marker in this study was very recently shown to be dysregulated in response to cerebral ischemia in mice (24). The authors found an increase of miR-181 in the ischemic core area of the brain and decreased levels in the penumbra. Apart from these associations we show here the dysregulation of distinct miRNAs in the course of acute myocardial infarction, which also suggests a potential active role in cardiovascular disease. Hence, studies in cellular and animal models of myocardial infarction will hopefully help to understand their functional role in the different stages and etiologies of MI.

The detailed pathophysiological role of the observed changes in miRNA expression remains unclear. Nevertheless it is likely that they are - at least in combination - rather specific for myocardial infarction. In a large multi-center, multi-disciplinary study we have recently shown that miRNA expression observed in AMI patients can be well discriminated to various other diseases (13). Since the here refined miRNA signature includes miRNAs that were previously identified in AMI and were subsequently compared with numerous other diseases, it can be assumed that they indeed reflect molecular mechanisms of AMI.

So far, most miRNA studies measured specific candidate miRNAs released from damaged myocardium into the serum of AMI patients. In a very recently published study, for example, Devaux et al. found miR-208b and miR-499 to be highly increased in serum samples of patients with AMI as determined by quantitative PCR (25). Wang and co-workers showed 4 muscle-enriched or cardiac specific miRNAs (miR-1, miR-133a, miR-499 and miR208a) to be up-regulated in plasma of AMI patients (26) with a decrement in circulating levels two months after myocardial infarction. However, the authors focused on a single measurement within the first 12 hours after the onset of symptoms (4.8 +/- 3.5h), when patients already showed highly elevated Troponin levels. In another report, the Capogrossi group found similar kinetics of cardiac miRNA and Troponin release into circulation (27). The authors assessed plasma levels of circulating miRNAs in a total of 41 AMI patients. In a subgroup of 8 patients, they performed serial measurements of both circulating miRNAs and Troponin from 156 minutes up to 69 hours after the onset symptoms, showing similar kinetics. In detail, they found miR-1, miR-133a and miR-133b to be up-regulated in AMI patients, with a peak level shortly before the peak of Troponin I, whereas miR-499-5p showed a slower time-course. Since the release of molecules from damaged myocardium into the blood stream may be similar for miRNAs and proteins (e.g. Troponins), miRNA assessment in serum or plasma might not lead to the development of earlier, and even more importantly more specific markers for AMI diagnosis. We hypothesised that a whole-blood approach (28) can result in a very early marker, reflecting disease processes involved in the early pathogenesis of AMI, such as plaque rupture, inflammation, coagulation and vascular injury (29, 30), rather than relying on cardiac necrosis. Hence, it is conceivable that expression profiles of miRNAs could also give insights into the underlying cause of an acute coronary syndrome in individual patients. The understanding of the mechanisms that led to myocardial infarction in the individual patient could also impact on therapeutic strategies and risk stratification. Thromboembolic myocardial infarction, for example, would warrant oral anticoagulation therapy, whereas rupture of atherosclerotic plaques would imply life-long medical treatment of cardiovascular risk factors.

Today, diagnosis of acute myocardial infarction relies on patients' symptoms, signs of ischemia in the electrocardiogram, and molecular biomarkers. Since symptoms might not be typical and electrocardiagraphical alterations are not specific in all cases, biomarkers have gained increasing importance and cardiac Troponins have become the gold-standard to detect acute myocardial necrosis. However, Troponins as single markers for AMI do have certain limitations, e.g. regarding their clinical specificity and early accessibility. MiRNAs could here facilitate advancements in clinical diagnostics by adding distinct properties to a multiple biomarker approach. The present work illustrates that such a multiple marker strategy can inter alia be based on miRNAs. Our study population is quite small, but incorporates 5 time-points of well-selected, early-stage AMI patients resulting in 35 whole-genome miRNA measurements, enabling detailed analyses of the dynamic disease miRNome. The advantage of the identified miRNA markers might be that that they do not depend on leakage from necrotic cardiomyocytes, but are derived from circulating blood cells involved in the pathophysiology of MI. However, larger, prospective trials will be needed to finally confirm the statistical value of these miRNAs as novel biomarkers for acute myocardial infarction.

**Table 1: Patient characteristics**

| Characteristics | Patients with AMI (n=7) | Patients without AMI (n=11) | *p*-value |
|---|---|---|---|
| Age (years) | 65 ± 11 | 61 ± 16 | 0.46 |
| Male/female*(n*/*n)* | 6/1 | 6/5 | 0.2 |
| DM, *n* (%) | 2 (29) | 4 (36) | 0.43 |
| Hypertension, *n (%)* | 6 (86) | 8 (72) | 0.11 |
| Current smoking, *n* (%) | 5 (72) | 3 (27) | 0.009 |
| Hyperlipidaemia, *n* (%) | 5 (72) | 6 (54) | 0.11 |
| SBP (mmHg) | 128 ± 23 | 125 ± 15 | 0.74 |
| DBP (mmHg) | 76 ± 12 | 77 ± 11 | 0.85 |
| TG (mmol/L) | 1,36 ± 0,4 | 1,91 ± 1,2 | 0.28 |
| HDL (mmol/L) | 0,96 ± 0,2 | 1,17 ± 0,6 | 0.5 |
| LDL (mmol/L) | 2,76 ± 1 | 2,52 ± 1 | 0.74 |
| WBC/nL | 13 ± 4 | 9.1 ± 3 | 0.07 |
| Creatinine (µmol/L) | 83,2 ± 17 | 81,4 ± 18 | 0.86 |
| Urea (mmol/L) | 5,18 ± 1,2 | 5,34 ± 2 | 0.88 |

| | | | |
|---|---|---|---|
| *DM* = diabetes mellitus; *SBP* = systolic blood pressure; *DBP* = diastolic blood pressure; *TG* = triglycerides; *HDL =* high density lipoprotein; *LDL* = low density lipoprotein; *WBC* = white blood cell count. | | | |

### References

1. Thygesen K, Alpert JS, White HD, Jaffe AS, Apple FS, Galvani M, et al. Universal definition of myocardial infarction. Circulation 2007;116:2634-53.
2. Katus HA, Remppis A, Looser S, Hallermeier K, Scheffold T, Kubler W. Enzyme linked immuno assay of cardiac Troponin t for the detection of acute myocardial infarction in patients. J Mol Cell Cardiol 1989;21:1349-53.
3. Celik S, Giannitsis E, Wollert KC, Schwobel K, Lossnitzer D, Hilbel T, et al. Cardiac Troponin t concentrations above the 99th percentile value as measured by a new high-sensitivity assay predict long-term prognosis in patients with acute coronary syndromes undergoing routine early invasive strategy. Clin Res Cardiol 2011.
4. Giannitsis E, Kurz K, Hallermayer K, Jarausch J, Jaffe AS, Katus HA. Analytical validation of a high-sensitivity cardiac Troponin t assay. Clin Chem 2010;56:254-61.
5. Hamm CW, Ravkilde J, Gerhardt W, Jorgensen P, Peheim E, Ljungdahl L, et al. The prognostic value of serum Troponin t in unstable angina. N Engl J Med 1992;327:146-50.
6. Lee RC, Feinbaum RL, Ambros V. The c. Elegans heterochronic gene lin-4 encodes small rnas with antisense complementarity to lin-14. Cell 1993;75:843-54.
7. Lee CT, Risom T, Strauss WM. Evolutionary conservation of microrna regulatory circuits:
   An examination of microrna gene complexity and conserved microrna-target interactions through metazoan phylogeny. DNA Cell Biol 2007;26:209-18.
8. Cai B, Pan Z, Lu Y. The roles of micrornas in heart diseases: A novel important regulator. Curr Med Chem 2010;17:407-11.
9. Dong S, Cheng Y, Yang J, Li J, Liu X, Wang X, et al. Microrna expression signature and the role of microrna-21 in the early phase of acute myocardial infarction. J Biol Chem 2009;284:29514-25.
10. Yang B, Lin H, Xiao J, Lu Y, Luo X, Li B, et al. The muscle-specific microrna mir-1 regulates cardiac arrhythmogenic potential by targeting gja1 and kcnj2. Nat Med 2007;13:486-91.
11. Terentyev D, Belevych AE, Terentyeva R, Martin MM, Malana GE, Kuhn DE, et al. Mir-1 overexpression enhances ca(2+) release and promotes cardiac arrhythmogenesis by targeting pp2a regulatory subunit b56alpha and causing camkii-dependent hyperphosphorylation of ryr2. Circ Res 2009;104:514-21.
12. Meder B, Katus HA, Rottbauer W. Right into the heart of microrna-133a. Genes Dev 2008;22:3227-31.
13. Keller A, Leidinger P, Bauer A, Elsharawy A, Haas J, Backes C, et al. Toward the blood-borne mirnome of human diseases. Nat Methods 2011.
14. Meder B, Keller A, Vogel B, Haas J, Sedaghat-Hamedani F, Kayvanpour E, et al. Microrna signatures in total peripheral blood as novel biomarkers for acute myocardial infarction. Basic Res Cardiol 2011;106:13-23.
15. Tijsen AJ, Creemers EE, Moerland PD, de Windt LJ, van der Wal AC, Kok WE, Pinto YM. Mir423-5p as a circulating biomarker for heart failure. Circ Res 2010;106:1035-9.
16. Vorwerk S, Ganter K, Cheng Y, Hoheisel J, Stahler PF, Beier M. Microfluidic-based enzymatic on-chip labeling of mirnas. N Biotechnol 2008;25:142-9.
17. Keller A, Leidinger P, Borries A, Wendschlag A, Wucherpfennig F, Scheffler M, et al. Mirnas in lung cancer - studying complex fingerprints in patient's blood cells by microarray experiments. BMC Cancer 2009;9:353.
18. Griffiths-Jones S, Saini HK, van Dongen S, Enright AJ. Mirbase: Tools for microrna genomics. Nucleic Acids Res 2008;36:D154-8.
19. Bolstad BM, Irizarry RA, Astrand M, Speed TP. A comparison of normalization methods for high density oligonucleotide array data based on variance and bias. Bioinformatics 2003;19:185-93.
20. Aliferis CF, Statnikov A, Tsamardinos I, Schildcrout JS, Shepherd BE, Harrell FE, Jr. Factors influencing the statistical power of complex data analysis protocols for molecular signature development from microarray data. PLoS ONE 2009;4:e4922.
21. Erdogan B, Facey C, Qualtieri J, Tedesco J, Rinker E, Isett RB, et al. Diagnostic micrornas in myelodysplastic syndrome. Exp Hematol 2011;39:915-26 e2.
22. Ujifuku K, Mitsutake N, Takakura S, Matsuse M, Saenko V, Suzuki K, et al. Mir-195, mir-455-3p and mir-10a(*) are implicated in acquired temozolomide resistance in glioblastoma multiforme cells. Cancer Lett 2010;296:241-8.
23. Xu K, Liang X, Cui D, Wu Y, Shi W, Liu J. Mir-1915 inhibits bcl-2 to modulate multidrug resistance by increasing drug-sensitivity in human colorectal carcinoma cells. Mol Carcinog 2011.
24. Ouyang YB, Lu Y, Yue S, Xu LJ, Xiong XX, White RE, et al. Mir-181 regulates grp78 and influences outcome from cerebral ischemia in vitro and in vivo. Neurobiol Dis 2012;45:555-63.
25. Devaux Y, Vausort M, Goretti E, Nazarov PV, Azuaje F, Gilson G, et al. Use of circulating micrornas to diagnose acute myocardial infarction. Clin Chem 2012;58:559-67.
26. Wang GK, Zhu JQ, Zhang JT, Li Q, Li Y, He J, et al. Circulating microrna: A novel potential biomarker for early diagnosis of acute myocardial infarction in humans. Eur Heart J 2010;31:659-66.
27. D'Alessandra Y, Devanna P, Limana F, Straino S, Di Carlo A, Brambilla PG, et al. Circulating micrornas are new and sensitive biomarkers of myocardial infarction. Eur Heart J 2010;published online June 9, 2010 doi:10.1093/eurheartj/ehq167.
28. Hausler SF, Keller A, Chandran PA, Ziegler K, Zipp K, Heuer S, et al. Whole blood-derived mirna profiles as potential new tools for ovarian cancer screening. Br J Cancer 2010;103:693-700.
29. Hoekstra M, van der Lans CA, Halvorsen B, Gullestad L, Kuiper J, Aukrust P, et al. The peripheral blood mononuclear cell microrna signature of coronary artery disease. Biochem Biophys Res Commun 2010;394:792-7.
30. Voellenkle C, van Rooij J, Cappuzzello C, Greco S, Arcelli D, Di Vito L, et al. Microrna signatures in peripheral blood mononuclear cells of chronic heart failure patients. Physiol Genomics 2010;42:420-6.

## Claims

1. A method for differential diagnosis of acute myocardial infarction, comprising the steps:
(a) determining the level of at least one miRNA with SEQ ID NO: 5 (hsa-miR-380*) in a blood cell test sample from a subject,
(b) comparing the level of said at least one miRNA of (a) with a reference level of said at least one miRNA,
(c) determining the level of cardiac troponin in a serum or plasma test sample from said subject, and
(d) comparing the level of cardiac troponin of (c) with a reference level of cardiac troponin,
wherein the comparison of step (b) together with the comparison of step (d) allows for differential diagnosis between acute myocardial infarction and diseases that give rise to cardiac troponin levels and that are not acute myocardial infarction,
wherein the cardiac troponin level being above the 99th percentile value of a reference population and the level of said at least one miRNA being not decreased by a factor of at least 1.5 results in the diagnosis of diseases that give rise to cardiac troponin levels and that are not acute myocardial infarction, wherein the cardiac troponin level being above the 99th percentile value of a reference population and the level of said at least one miRNA being decreased by a factor of at least 1.3 results in the diagnosis of acute myocardial infarction, or
wherein the cardiac troponin level being not above the 99th percentile value of a reference population and the level of said at least one miRNA being decreased by a factor of at least 1.3 results in the diagnosis of acute myocardial infarction.

2. The method of claim 1, wherein the reference level of (b) is determined from subjects not suffering from acute myocardial infarction.

3. The method of any of the claims 1 to 2, wherein the cardiac troponin level being above 14 pg/ml, more preferably above 50 pg/ml, and the level of said at least one miRNA being not decreased by a factor of at least 1.3, results in the diagnosis of diseases that give rise to cardiac troponin levels and that are not acute myocardial infarction.

4. The method of claim 3, wherein said diseases are selected from heart failure, cardiomyopathy, myocarditis or pulmonary embolism, or (myo)pericarditis, (left) ventricular hypertrophy, peripartum cardiomyopathy, Takotsubo cardiomyopathy, cardiac amyloidosis, non-ishemic (systolic) heart failure.

5. The method of any of the claims 1 to 2, wherein the differential diagnosis allows for exclusion of diseases that give rise to a cardiac troponin level and which are not acute myocardial infarction.

6. The method of claims 5, wherein the differential diagnosis allows for exclusion of heart failure, cardiomyopathy, myocarditis or pulmonary embolism, or (myo)pericarditis, (left) ventricular hypertrophy, peripartum cardiomyopathy, Takotsubo cardiomyopathy, cardiac amyloidosis, non-ishemic (systolic) heart failure.

7. The method of any of the claims 1 to 2, wherein the cardiac troponin level being above 14 pg/ml, more preferably above 50 pg/ml, and the level of said at least one miRNA being decreased by a factor of at least 1.5 results in the diagnosis of acute myocardial infarction.

8. The method of any of the claims 1 to 2, wherein the cardiac troponin level being not above 50 pg/ml, more preferably not above 14 pg/ml, and the level of said at least one miRNA being decreased by a factor of at least 1.5 results in the diagnosis of acute myocardial infarction.

9. The method of any of the claims 1 to 8, wherein the at least one miRNA are at least 2 miRNAs selected from one or more sets listed in Figure 7, preferably selected from SMI-3, SMI-5, SMI-11, SMI-25, SMI-29, SMI-35, SMI-48, SMI-50, SMI-51, SMI-61.

10. The method of any of the claim 1 to 8, wherein further miRNAs are selected from the group consisting of SEQ ID NO: 2 (hsa-miR-566), 3 (hsa-miR-455-3p) or 7 (hsa-miR-1291).

11. Use of Cardiac troponin in early diagnosis of acute myocardial infarction in subjects with chest pain **characterized by** a cardiac troponin level being above the 99th percentile value of a reference population, preferably above 14pg/ml, more preferably above 50 pg/ml and by a not decreased level of a factor of at least 1.5, preferably by a not decreased level of a factor of at least 1.3 of at least one miRNA in a blood cell test sample from said subject, wherein said at least one miRNA is selected from SEQ ID NO: 5 (hsa-miR-380*).

## Patentansprüche

1. Verfahren zur Differentialdiagnose eines akuten Myokardinfarkts, die folgenden Schritte umfassend:
(a) Bestimmen des Levels wenigstens einer miRNA mit SEQ ID NO: 5 (hsa-miR-380*) in einer Blutzelltestprobe eines Subjekts;
(b) Vergleichen des Levels der wenigstens einen miRNA aus (a) mit einem Referenzlevel der wenigstens einen miRNA;
(c) Bestimmen des Levels von kardialem Troponin in einer Serum- oder Plasmatestprobe des Subjekts; und
(d) Vergleichen des Levels von kardialem Troponin aus (c) mit einem Referenzlevel von kardialem Troponin,
wobei der Vergleich aus Schritt (b) gemeinsam mit dem Vergleich aus Schritt (d) eine Differentialdiagnose zwischen akutem Myokardinfarkt und Erkrankungen ermöglicht, die mit Leveln von kardialem Troponin assoziiert sind und die kein akuter Myokardinfarkt sind;
wobei der Level von kardialem Troponin über der 99. Perzentile einer Referenzgruppe und der Level der wenigstens einen miRNA, der nicht um einen Faktor von mindestens 1,5 herabgesetzt ist, zur Diagnose von Erkrankungen führen, die mit Leveln von kardialem Troponin assoziiert sind und die kein akuter Myokardinfarkt sind;
wobei der Level von kardialem Troponin über der 99. Perzentile einer Referenzgruppe und der Level der wenigstens einen miRNA, der um einen Faktor von mindestens 1,3 herabgesetzt ist, zur Diagnose von akutem Myokardinfarkt führen; oder
wobei der Level von kardialem Troponin nicht über der 99. Perzentile einer Referenzgruppe und der Level der wenigstens einen miRNA, der um einen Faktor von mindestens 1,3 herabgesetzt ist, zur Diagnose von akutem Myokardinfarkt führen.

2. Verfahren nach Anspruch 1, wobei der Referenzlevel aus (b) an Subjekten bestimmt wird, die nicht an einem akuten Myokardinfarkt leiden.

3. Verfahren nach einem der Ansprüche 1 bis 2, wobei der Level von kardialem Troponin höher als 14 pg/ml, vorzugsweise höher als 50 pg/ml, und der Level der wenigstens einen miRNA, der nicht um einen Faktor von mindestens 1,3 herabgesetzt ist, zur Diagnose von Erkrankungen führen, die mit Leveln von kardialem Troponin assoziiert sind und die kein akuter Myokardinfarkt sind.

4. Verfahren nach Anspruch 3, wobei die Erkrankungen ausgewählt sind aus Herzinsuffizienz, Kardiomyopathie, Myokarditis oder Lungenembolie oder (Myo)perikarditis, (links)ventrikulärer Hypertrophie, peripartaler Kardiomyopathie, Takotsubo-Kardiomyopathie, kardialer Amyloidose, nicht-ischämischer (systolischer) Herzinsuffizienz.

5. Verfahren nach einem der Ansprüche 1 bis 2, wobei die Differentialdiagnose den Ausschluss von Erkrankungen ermöglicht, die mit einem Level von kardialem Troponin assoziiert sind und die kein akuter Myokardinfarkt sind.

6. Verfahren nach Anspruch 5, wobei die Differentialdiagnose den Ausschluss von Herzinsuffizienz, Kardiomyopathie, Myokarditis oder Lungenembolie oder (Myo)perikarditis, (links)ventrikulärer Hypertrophie, peripartaler Kardiomyopathie, Takotsubo-Kardiomyopathie, kardialer Amyloidose, nicht-ischämischer (systolischer) Herzinsuffizienz ermöglicht.

7. Verfahren nach einem der Ansprüche 1 bis 2, wobei der Level von kardialem Troponin höher als 14 pg/ml, vorzugsweise höher als 50 pg/ml, und der Level der wenigstens einen miRNA, der um einen Faktor von mindestens 1,5 herabgesetzt ist, zur Diagnose von akutem Myokardinfarkt führen.

8. Verfahren nach einem der Ansprüche 1 oder 2, wobei der Level von kardialem Troponin nicht höher als 50 pg/ml, vorzugsweise nicht höher als 14 pg/ml, und der Level der wenigstens einen miRNA, der um einen Faktor von mindestens 1,5 herabgesetzt ist, zur Diagnose von akutem Myokardinfarkt führen.

9. Verfahren nach einem der Ansprüche 1 bis 8, wobei die wenigstens eine miRNA wenigstens zwei miRNAs sind, die aus einem oder mehreren in Figur 7 aufgeführten Set(s) ausgewählt sind, wobei sie vorzugsweise aus SMI-3, SMI-5, SMI-11, SMI-25, SMI-29, SMI-35, SMI-48, SMI-50, SMI-51, SMI-61 ausgewählt sind.

10. Verfahren nach einem der Ansprüche 1 bis 8, wobei weitere miRNAs aus der Gruppe bestehend aus SEQ ID NO: 2 (hsa-miR-566), 3 (hsa-miR-455-3p) oder 7 (hsa-miR-1291) ausgewählt sind.

11. Verwendung von kardialem Troponin in der Früherkennung von akutem Myokardinfarkt bei Subjekten mit Brustschmerzen, **gekennzeichnet durch** einen Level von kardialem Troponin über der 99. Perzentile einer Referenzgruppe, bevorzugt über 14 pg/ml, bevorzugter über 50 pg/ml, und
durch einen nicht um mindestens Faktor 1,5 herabgesetzten Level, bevorzugt durch einen nicht um mindestens Faktor 1,3 herabgesetzten Level,
wenigstens einer miRNA in einer Blutzelltestprobe des Subjekts, wobei die wenigstens eine miRNA aus SEQ ID NO: 5 (hsa-miR-380*) ausgewählt ist.

## Revendications

1. Méthode de diagnostic différentiel d'un infarctus aigu du myocarde, comprenant les étapes de :
(a) détermination du taux d'au moins un miARN doté de la SEQ ID n° : 5 (hsa-miR-380*) dans un échantillon test de cellules sanguines provenant d'un sujet,
(b) comparaison du taux dudit au moins un miARN de (a) à un taux de référence dudit au moins un miARN,
(c) détermination du taux de troponine cardiaque dans un échantillon test de sérum ou de plasma provenant dudit sujet, et
(d) comparaison du taux de troponine cardiaque de (c) à un taux de référence de troponine cardiaque,
ladite comparaison de l'étape (b) avec la comparaison de l'étape (d) permettant un diagnostic différentiel entre un infarctus aigu du myocarde et des maladies qui sont à l'origine de taux de troponine cardiaque et qui ne sont pas un infarctus aigu du myocarde,
ledit taux de troponine cardiaque étant supérieure à la valeur au 99^{ème} percentile d'une population de référence et ledit taux dudit au moins un miARN n'étant pas diminué d'un facteur d'au moins 1,5 conduisant au diagnostic de maladies qui sont à l'origine de taux de troponine cardiaque et qui ne sont pas un infarctus aigu du myocarde,
ledit taux de troponine cardiaque étant supérieur à la valeur au 99^{ème} percentile d'une population de référence et ledit taux dudit au moins un miARN étant diminué d'un facteur d'au moins 1,3 conduisant au diagnostic d'un infarctus aigu du myocarde, ou
ledit taux de troponine cardiaque n'étant pas supérieur à la valeur au 99^{ème} percentile d'une population de référence et ledit taux d'au moins un miARN étant diminué d'un facteur d'au moins 1,3 conduisant au diagnostic d'un infarctus aigu du myocarde.

2. Méthode selon la revendication 1, ledit taux de référence de (b) étant déterminé à partir de sujets ne souffrant pas d'un infarctus aigu du myocarde.

3. Méthode selon l'une quelconque des revendications 1 à 2, ledit taux de troponine cardiaque étant supérieur à 14 pg/ml, plus préférablement supérieur à 50 pg/ml, et ledit taux dudit au moins un miARN étant non diminué d'un facteur d'au moins 1,3, conduisant au diagnostic de maladies qui sont à l'origine de taux de troponine cardiaque et qui ne sont pas un infarctus aigu du myocarde.

4. Méthode selon la revendication 3, lesdites maladies étant choisies parmi une insuffisance cardiaque, une cardiomyopathie, une myocardite ou une embolie pulmonaire, ou une (myo)péricardite, une hypertrophie ventriculaire (gauche), une cardiomyopathie du péripartum, la cardiomyopathie de Takotsubo, une amyloïdose cardiaque, une insuffisance cardiaque (systolique) non ischémique.

5. Méthode selon l'une quelconque des revendications 1 à 2, ledit diagnostic différentiel permettant l'exclusion de maladies qui sont à l'origine d'un taux de troponine cardiaque et qui ne sont pas un infarctus aigu du myocarde.

6. Méthode selon la revendication 5, ledit diagnostic différentiel permettant l'exclusion d'une insuffisance cardiaque, d'une cardiomyopathie, d'une myocardite ou d'une embolie pulmonaire, ou d'une (myo)péricardite, d'une hypertrophie ventriculaire (gauche), d'une cardiomyopathie du péripartum, de la cardiomyopathie de Takotsubo, d'une amyloïdose cardiaque, d'une insuffisance cardiaque (systolique) non ischémique.

7. Méthode selon l'une quelconque des revendications 1 à 2, ledit taux de troponine cardiaque étant supérieur à 14 pg/ml, plus préférablement supérieur à 50 pg/ml, et ledit taux dudit au moins un miARN étant diminué d'un facteur d'au moins 1,5 conduisant au diagnostic d'un infarctus aigu du myocarde.

8. Méthode selon l'une quelconque des revendications 1 à 2, ledit taux de troponine cardiaque n'étant pas supérieur à 50 pg/ml, plus préférablement pas supérieur à 14 pg/ml, et ledit taux dudit au moins un miARN étant diminué d'un facteur d'au moins 1,5 conduisant au diagnostic d'un infarctus aigu du myocarde.

9. Méthode selon l'une quelconque des revendications 1 à 8, ledit au moins un miARN étant au moins 2 miARN choisis parmi un ou plusieurs ensembles énumérés dans la figure 7, de préférence choisis parmi SMI-3, SMI-5, SMI-11, SMI-25, SMI-29, SMI-35, SMI-48, SMI-50, SMI-51, SMI-61.

10. Méthode selon l'une quelconque des revendications 1 à 8, dans laquelle des miARN supplémentaires sont choisis dans le groupe constitué par la SEQ ID n° : 2 (hsa-miR-566), 3 (hsa-miR-455-3p) ou 7 (hsa-miR-1291).

11. Utilisation de troponine cardiaque dans le diagnostic précoce d'un infarctus aigu du myocarde chez des sujets atteints de douleur à la poitrine **caractérisée par** un taux de troponine cardiaque étant supérieure à la valeur au 99^{ème} percentile d'une population de référence, de préférence supérieure à 14 pg/ml, plus préférablement supérieure à 50 pg/ml et par un taux non diminué d'un facteur d'au moins 1,5, de préférence par un taux non diminué d'un facteur d'au moins 1,3 d'au moins un miARN dans un échantillon test de cellules sanguines provenant dudit sujet, ledit au moins un miARN étant choisi parmi la SEQ ID n° : 5 (hsa-miR-380*).
